# EUROPEAN PATENT APPLICATION

(11) **EP 1 447 443 A1**
(43) Date of publication of application: **18.08.2004**
(21) Application number: 02775465.4
(22) Date of filing: 31.10.2002
(51) Int. Cl.: C12N 5/06, C12N 5/10, A61K 35/32, A61K 35/18, C12N 15/09

(54) **IMMORTALIZED MESENCHYMAL CELLS AND UTILIZATION THEREOF**

(30) Priority: 31.10.2001 JP 2001335375
(71) Applicant: Renomedix Institute Inc., Sapporo-shi, Hokkaido 060-0031 (JP); Hitachi Ltd., Tokyo 101-8010 (JP); Mitsui Sumitomo Insurance Care Network Company, Limited, Tokyo 101-0052 (JP); Teikoku Hormone Mfg. Co., Ltd., Tokyo 108-8532 (JP)
(72) Inventor: HAMADA, Hirofumi, Sapporo-shi, Hokkaido 064-0959 (JP); KAWANO, Yutaka, Chuo-ku, Sapporo-shi, Hokkaido 060-0061 (JP); NAKAMURA, Kiminori, Sapporo-shi, Hokkaido 005-0012 (JP); KOBUNE, Masayoshi, Sapporo-shi, Hokkaido 064-0916 (JP); HONMOU, Osamu, Chuo-ku, Sapporo-shi, Hokkaido 064-0801 (JP); TANOOKA, Atsushi, Asahikawa-shi, Hokkaido 078-8215 (JP); OKA, Shin-ichi, Chuo-ku, Sapporo-shi, Hokkaido 064-0003 (JP); SASAKI, Katsunori, Sapporo-shi, Hokkaido 065-0032 (JP); TSUDA, Hajime, Sapporo-shi, Hokkaido 064-0809 (JP); ITO, Yoshinori, Sapporo-shi, Hokkaido 064-0921 (JP); KATO, Junji, Sapporo-shi, Hokkaido 064-0917 (JP); MATSUNAGA, Takuya, Sapporo-shi, Hokkaido 003-0003 (JP); NIITSU, Yoshiro, Sapporo-shi, Hokkaido 064-0823 (JP)
(74) Representative: Denison, Christopher Marcus
(86) International application number: PCT/JP2002/011389
(87) International publication number: WO 2003/038076

(57) **Abstract**

The method developed herein is for expanding cord blood-derived hematopoietic stem cells to a degree that is sufficiently safe for clinical application, such as the transplantation of hematopoietic stem cells into adult patients. Further, to prepare a number of mesenchymal stem cells or mesenchymal cells that have conventionally been available only in an extremely small number, an immortalizing gene such as that of telomerase is introduced alone into mesenchymal stem cells, mesenchymal cells or the like, so as to induce the differentiation of the expanded mesenchymal stem cells.

## Description

### TECHNICAL FIELD

The present invention relates to a method for expanding cells, and in particular to a method for expanding stem cells that have been difficult to obtain in a sufficient quantity.

Further, the present invention also relates to a method for utilizing the thus expanded cells. The present invention also relates to regeneration medicine using the thus expanded cells.

### BACKGROUND ART

Transplantation of hematopoietic stem cells that is performed for intractable blood diseases such as leukemia requires a large quantity of blood stem cells. Hematopoietic stem cells are undifferentiated cells that differentiate into blood components including leukocytes, erythrocytes and platelets. Hematopoietic stem cells can be collected from bone marrow, peripheral blood or cord blood by bone marrow biopsy, peripheral blood stem cell collection or the like.

To date, hematopoietic stem cells have been used to treat tumors and hematologic disorders. Cord blood is used as a supply source of blood stem cells, instead of stem cells from bone marrow. However, one problem is that it is difficult to obtain the blood stem cells of cord blood in a number sufficient for treatment.

Therefore, in vitro expansion of hematopoietic stem cells has been attempted. For example, Dick et al. succeeded in expanding hematopoietic stem cells by 2- to 4-fold within 4 days in the presence of stem cell factor (SCF), fit 3 ligand (FL), granulocyte colony-stimulating factor (G-CSF), interleukin-3 (IL-3) and interleukin-6 (IL-6) and even in the absence of stromal cells. However, in a longer culture, reduced stem cell activity was observed (J Exp Med 186: 619). Meanwhile, Eaves et al. reported that they could achieve a 3-fold expansion of murine stem cells using a stromal cell-free culture system supplemented with interleukin 11, FL and SCF, and a 2-fold expansion of human stem cells by stimulating the stem cells with SCF, FL, G-CSF, IL-3 and IL-6 (Proc. Natl. Acad. Sci. USA vol. 94, 13648).

Recently, it is increasingly understood that contact between stromal cells and the sub-cultured cells is important in maintaining the totipotency of stem cells. For example, expansion of hematopoietic stem cells was attempted by means of in vitro coculture with supporting cells (stroma cell or stromal cell) and various cytokines, so that expansion was achieved to some extent, but this was insufficient for clinical application (Experimental Hematology 29, 174-182).

Further, the involvement of telomerase in canceration of cells has been suggested. Accordingly, cell immortalization has been attempted by introducing a telomerase gene in combination with an oncogene such as ras. Although cell immortalization has been attempted by introducing telomerase into several types of cells, none of these attempts have succeeded in cell immortalization while keeping the normal cell functions without canceration (Nature Vol. 400 p. 465).

### DISCLOSURE OF THE INVENTION

### Objects to be achieved by the Invention

A first object of the present invention is to develop a method for expanding cord blood-derived hematopoietic stem cells to a degree sufficiently safe for clinical applications such as hematopoietic stem cell transplantation into adult patients.

Currently, erythrocytes of the same type provided by blood donation are mainly used as a source of erythrocyte transfusion for patients with anemia. However, problems exist, such as a shortage of blood cell donors with rare blood types and possible inoculation of an unknown source of infection. A second object of the present invention is to establish a safe supply source of erythrocytes in large quantities by amplifying hematopoietic stem cells using immortalized stromal cells, and then preparing a system that induces production of erythroid precursor cells at a high rate.

A third object of the present invention is to develop an artificial culture product that can expand hematopoietic stem cells stably for the long term together with the supporting cells, that is, to develop artificial bone marrow.

Furthermore, an object of the present invention is, in regeneration of various tissues and organs, to artificially regenerate, from tissues other than fetal cells, tissues such as cardiovascular tissues or nerve tissues that are effective for treatment.

### Means to achieve the objectives

First we introduced a virus vector having an oncogene or an immortalizing gene such as telomerase incorporated therein into a stromal cell in vitro. As a result, we have found that cell expansion is maintained after repeated cell division when telomerase alone has been introduced. Specifically, we have found that although the cell lifespan is drastically extended, the cell shape remains the same as that of a normal cell, and the cells can be used as expansion-supporting cells for blood precursor cells in a manner similar to that for normal cells.

By the use of the immortalized stromal cells having drastically extended lifespans as supporting cells for hematopoietic precursor cells that are obtained from cord blood or the like, expansion of hematopoietic precursor cells can be surprisingly enhanced. Further, production of erythroid precursor cells is induced by the use of the thus expanded hematopoietic precursor cell line, so that a system for supplying erythrocytes in large quantities for patients with anemia or the like can be developed without fear of unknown infection.

Moreover, we have also found that by the introduction of an immortalizing gene alone such as telomerase not only into a stromal cell but also into a mesenchymal stem cell, the mesenchymal stem cell can also be immortalized without losing its properties as a stem cell, such that the cell can induce cell differentiation when appropriate conditions are employed for inducing differentiation. We have then found that not only stem cells, but also the precursor cells of mesenchymal cells that can differentiate into mesenchymal cells and cells that are derived from mesenchymal cells can also be immortalized similarly, and thus we have completed the present invention.

Furthermore, we have found that when the thus immortalized mesenchymal stem cells, mesenchymal precursor cells, and mesenchymal cells are allowed to coexist with cells of various tissues, these tissues can be expanded and an artificial tissue construct can be established.

### BRIEF DESCRIPTION OF DRAWINGS

Fig. 1 shows the vector structures to be used for gene transfer into stromal cells.
Fig. 2 shows transfection into stromal cells.
Fig. 3 shows the number of generation after stromal cell division (primary, and hTERT, SV40T and ras introduction).
Fig. 4 shows the number of generation after stromal cell division (primary, and SV40T, ras, and SV40T/ras introduction).
Fig. 5 shows the number of generation after stromal cell division (hTERT, and hTERT/ras, hTERT/SV40T, and hTERT/SV40T/ras introduction).
Fig. 6 shows May-Giemsa staining of primary stromal cells and hTERT-transduced stromal cells.
Fig. 7 shows May-Giemsa staining of primary stromal cells and various genes-transduced stromal cells.
Fig. 8 shows telomerase activity.
   Lane 1. Molecular weight marker
   Lane 2. Negative control
   Lane 3. Heat sample of 5.
   Lane 4. Primary stromal cells
   Lane 5. hTERT-stromal cells
   Lane 6. Hela cells
Fig. 9 shows expression analysis of cell surface antigens of NK-derived stromal cells.
Fig. 10 shows expression analysis of cell surface antigens of KY-derived stromal cells.
Fig. 11 shows mRNA expression of cytokines.
Fig. 12 shows cell expansion after coculture of CD34+cord blood cells with stromal cells.
Fig. 13 shows cell expansion after coculture of CD34+cord blood cells with stromal cells.
Fig. 14 shows cell expansion after coculture of CD34+cord blood cells with stromal cells.
Fig. 15 shows cell expansion after coculture of CD34+cord blood cells with stromal cells.
Fig. 16 shows the number of generation after cell division of mesenchymal stem cells.
Fig. 17 shows induction of differentiation of mesenchymal stem cells.
Fig. 18 shows CD34+cord blood cells with hTERT-transduced mesenchymal stem cells or stromal cells.
Fig. 19 shows culturing of erythroblasts.
Fig. 20 shows induction of differentiation into erythroblasts after 14 days of coculture in the amplification phase of stem cells.
Fig. 21 shows culturing for differentiation into erythroblasts after 28 days of coculture in the amplification phase of stem cells.
Fig. 22 shows May-Giemsa staining of erythroblasts that have been induced to differentiate.
Fig. 23 shows the total cell number after differentiation of CD34+ cells into mature erythrocytes using immortalized stromal cells.
Fig. 24 shows the proportion of Glycophorin A positive cells as analyzed by FACS.
Fig. 25 shows the differentiation into and the expansion of erythroblastic cells, and the number of Glycophorin A positive cells.
Fig. 26 shows the results of May-Giemsa (MG) staining.
Fig. 27 shows the number of mature erythrocytes.
Fig. 28 shows images of May-Giemsa staining on day 28 (erythroblastic cells, basophilic erythroblasts, and polychromatic erythroblasts are mainly shown).
Fig. 29 shows images of May-Giemsa staining on day 31 (polychromatic erythroblasts are mainly shown, and enucleated mature erythrocytes are also shown).
Fig. 30 shows images of May-Giemsa staining on day 31 (macrophages surround an erythroblastic cell so as to form erythroblastic islands).
Fig. 31 shows the analysis of hCD45+cells in bone marrow and peripheral blood of a transplanted NOD/SCID mouse.
Fig. 31A shows hCD45+ cells in bone marrow as analyzed by flow cytometry.
Fig. 31C shows hCD45+ cells in peripheral blood as analyzed by flow cytometry.
Fig. 31A and C
   X: accessory cells; □, pre-coculture with CD 34+ cells; Δ, CD 34+ cells expanded ex vivo for 2 weeks without stromal cells; ? , CD 34+ cells expanded on primary stromal cells for 2 or 4 weeks; ? , CD 34+ cells expanded using hTERT- stromal cells for 2 or 4 weeks.
   A dotted line shows the cutoff (0.1 %) of successful transplantation of human hematopoietic cells.
   p *<0.05 (by Mann-Whiteney U test) compared with pre-cocultured CD 34+ cells.

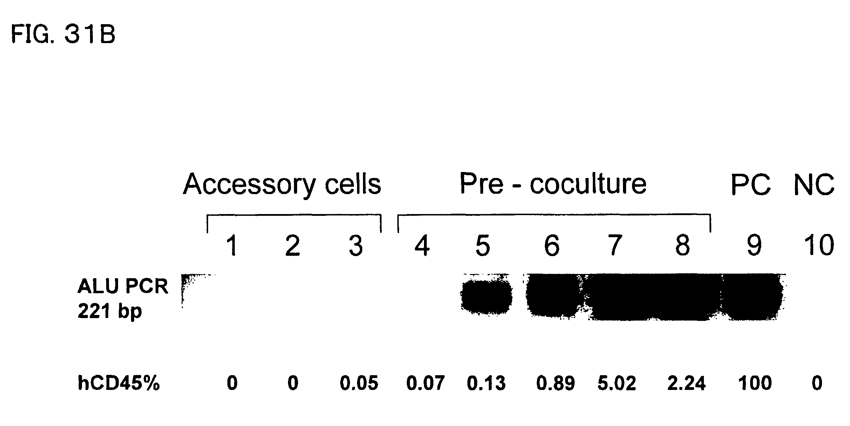

Fig. 31B shows the PCR amplification of human alu sequence and the proportion of hCD45 cells in the bone marrow of a transplanted NOD/SCID mouse.
   Lanes 1-3, mice (3) transplanted with only accessory cells.
   Lanes 4-8, mice (5) transplanted with pre-cocultured CD34+ cells.
   Lane 9, PC (positive control, and human peripheral blood monocytes).
   Lane 10, NC (a non-transplanted, negative control mouse (1)).

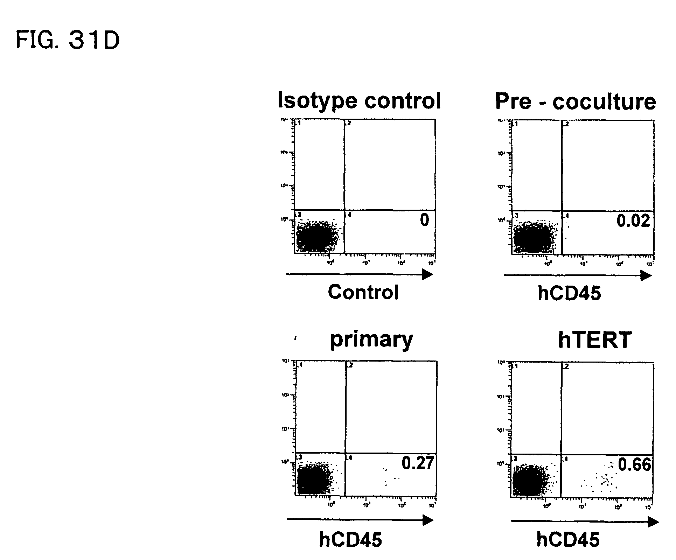

Fig. 31D shows the peripheral blood of NOD/SCID mice as analyzed by flow cytometry using an anti-human CD 45 antibody.
   The figure shows analytical results when pre-coculured CD34+ cells (upper right), hematopoietic cells expanded for 4 weeks on primary stromal cells (lower left), or hematopoietic cells expanded for 4 weeks on h-TERT stromal cells (lower right) were transplanted to NOD/SCID mice. Data obtained using an isotype match antibody against a monocyte of the peripheral blood of the transplanted mouse are also shown (upper left).
   The Y-axis shows staining with PI (propidium iodide).

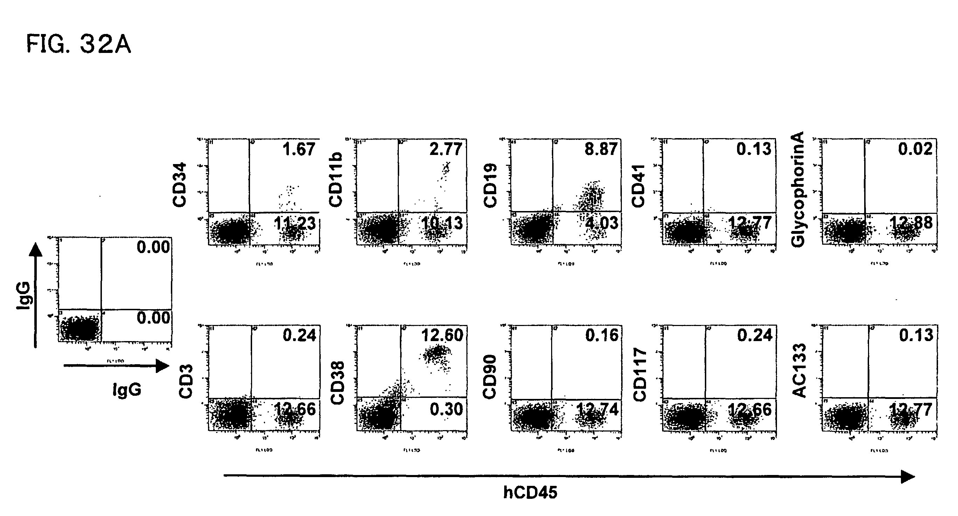

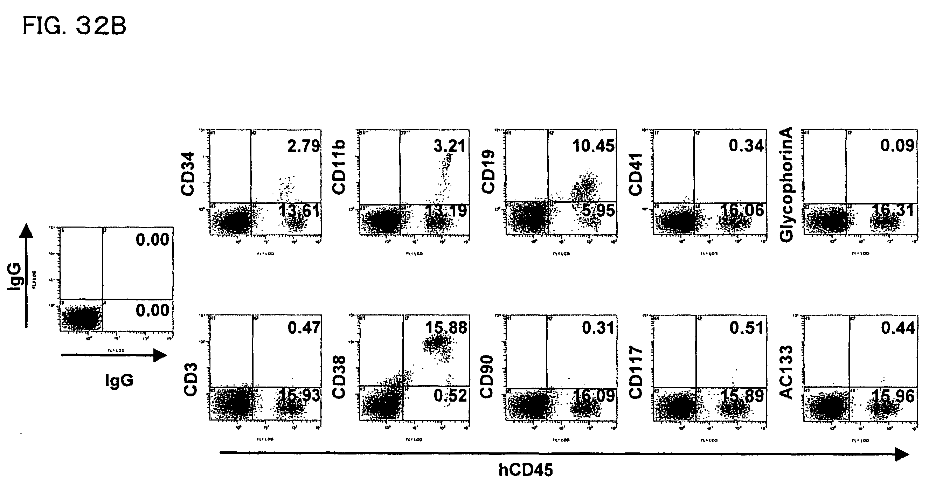

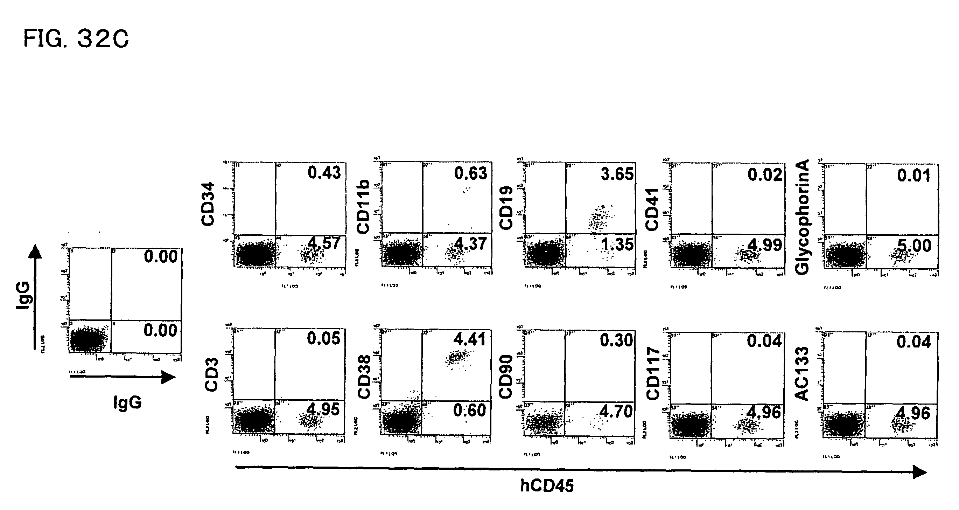

Fig. 32 shows the lineage marker of human hematopoietic cells that have been transplanted into NOD/SCID mice as analyzed by flow cytometry.
   CD34+ cells were transplanted into NOD/SCID mice after (A) 4 weeks of expansion on primary stromal cells, (B) 4 weeks of expansion on hTERT-stromal cells or (C) without expansion (pre-coculture). To confirm human origination, hematopoietic cells were immuno-labeled with FITC-conjugated hCD45 antibodies, and further immuno-labeled with PE-conjugated antibodies specific to the indicated lineage markers.

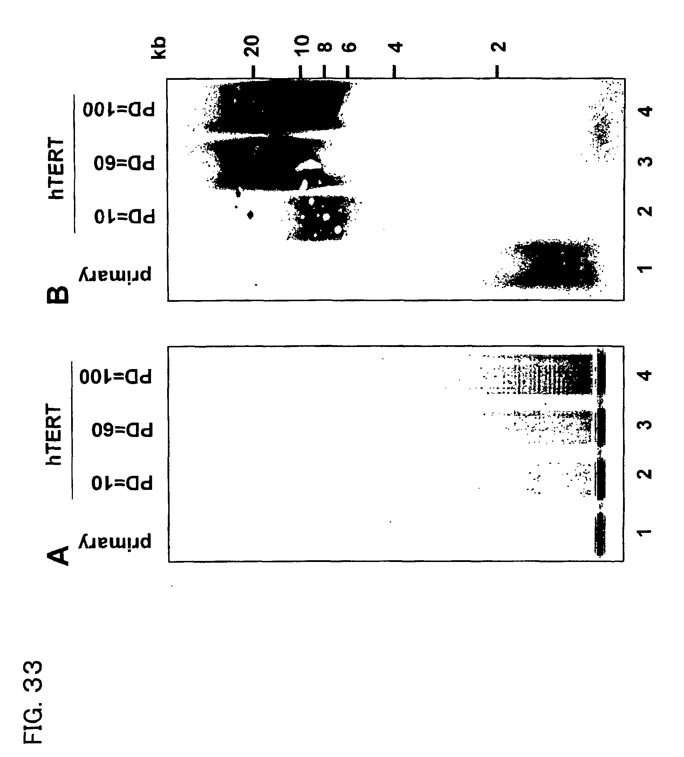

Fig. 33 shows the telomerase activity and telomere length of primary stromal cells or hTERT stromal cells.
Fig. 33(A) Telomerase activity
   Lane 1, primary stromal cells; lane 2, h-TERT stromal cells (PD=10); lane 3, h-TERT stromal cells (PD=60); Lane 4, h-TERT stromal cells (PD=100) 9
Fig. 33(B) Telomere length
   Lane 1, primary stromal cells; lane 2, h-TERT stromal cells (PD=10); lane 3, h-TERT stromal cells (PD=60); Lane 4, h-TERT stromal cells (PD=100)

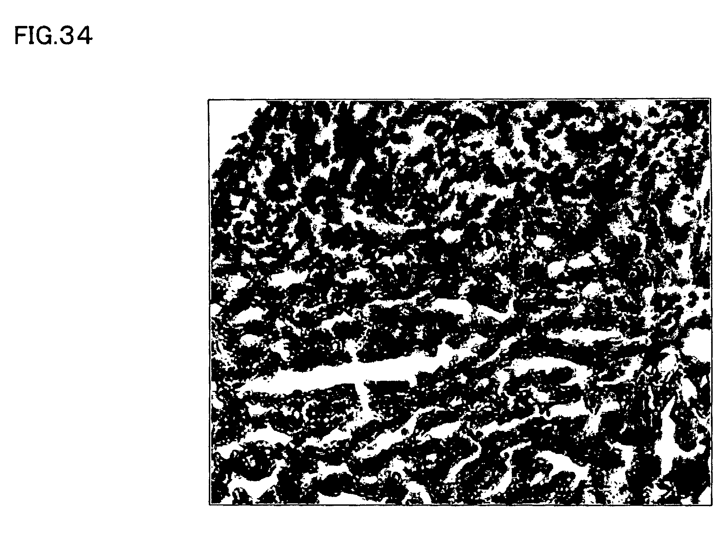

Fig. 34 shows MSC-transplanted rat hepatocytes stained using anti-human albumin antibodies (Sigma, A6684).

### BEST MODE FOR CARRYING OUT THE INVENTION

The present invention encompasses: (1) a method for cell immortalization, which involves introducing immortalizing genes into mesenchymal stem cells, mesenchymal precursor cells, mesenchymal cells or cells derived from mesenchymal cells (hereinafter, referred to as mesenchymal cell-related cells) to immortalize the cells, and the thus immortalized cells; (2) cells that are differentiated from the immortalized mesenchymal stem cells and a method for differentiating the same; (3) a method for long-term cell expansion, which involves introducing immortalizing genes into stromal cells when hematopoietic stem cells (precursor cells) are cocultured with stromal cells; (4) a method for long-term cell expansion and a method for regulating cell differentiation, which involve introducing immortalizing genes into mesenchymal cells when cardiovascular cells are cocultured with mesenchymal cells; (5) a method for long-term cell expansion, and a method for regulating cell differentiation, which involve introducing immortalizing genes into mesenchymal cells, when bone, cartilage, tendon, skeletal muscle, adipose tissue or the like is cocultured with mesenchymal cells; (6) a method for long-term cell expansion, and a method for regulating cell differentiation, which involve introducing immortalizing genes into mesenchymal cells when neural cells are cocultured with mesenchymal cells; (7) a method for long-term cell expansion, and a method for regulating cell differentiation, which involve introducing immortalizing genes into mesenchymal cells when endocrine cells are cocultured with mesenchymal cells; and (8) an examination method and a therapy using immortalized cells or cells subjected to modification such as expansion or differentiation using immortalized cells.

The present invention further encompasses an in vitro assay using immortalized, mesenchymal system-related cells, preferably an in vitro assay which is an examination method for evaluating drug efficacy and a kit for this in vitro assay. The present invention further encompasses methods for analyzing the pathological conditions of diseases, diagnostic methods, therapies, blood transfusion therapies, therapies for the cardiovascular system, therapies for bone, cartilage, tendon, skeletal muscle, and adipose tissue, therapies for nervous diseases, therapies for endocrine diseases, therapies for ischemic heart diseases and arteriosclerosis obliterans, therapies for osteoarthritis, rheumatic arthropathy, injury, intractable bone/cartilage defects, and therapies for neurodegenerative diseases, dementia, cerebrovascular disorder and nerve injury.

Immortalized cells (cell immortalization) in the present invention refer to cells that can continuously expand even after cell division has been repeated a certain number of times. Normal cells cease their expansion after repeating cell division for a particular number of times.

An immortalizing gene in the present invention refers to a telomerase or a gene that regulates the expression or the activity of telomerase. Preferably, a human telomerase can be used. Further, for example, the myc gene is said to enhance telomerase activity.

Any known various methods can be used for introducing immortalizing genes into mesenchymal system-related cells such as stromal cells or mesenchymal stem cells. Examples of such methods that can be used herein include a transformation method, which incorporates an immortalizing gene into a plasmid vector, introducing the vector into mesenchymal cells such as stromal cells, mesenchymal stem cells or the like in the presence of calcium-phosphate; an introduction method, which introduces an immortalizing gene together with a liposome-like vesicle into mesenchymal cells or mesenchymal stem cells through contact with these cells; an introduction method, which involves eletroporation in the presence of immortalizing genes; and an introduction method, which incorporates immortalizing genes into various virus vectors, and allowing mesenchymal cells, mesenchymal stem cells or the like to be infected with these virus vectors.

Examples of introduction methods using a virus vector include methods using a retrovirus, an adenovirus, or an adeno-associated virus. One example of such a method uses the MoMLV virus as a retrovirus vector. Preferably, the pBabe vector can be used.

Furthermore, when immortalized cells are returned into a patient, it is considered safer to previously remove a foreign gene such as an immortalizing gene or the like insofar as is possible. Such an immortalizing gene that has been introduced into a cell can be removed by previously established techniques. For example, preferably, a technique that can be used herein involves specifically removing an immortalizing gene placed between loxP sequences or loxP-like sequences by treatment with recombinase such as Cre recombinase.

The mesenchymal system-related cell refers to mesenchymal stem cells, mesenchymal cells, precursor cells of mesenchymal cells or a cell that is derived from mesenchymal cells.

The mesenchymal stem cell refers to, for example, a stem cell that can be obtained from bone marrow, peripheral blood, skin, hair root, muscle tissue, endometrium, blood, or cord blood, and also from the product of primary culturing of various tissues. Further, it is also known that the mesenchymal stem cells can also be isolated from ES cells or teratoma cells. Examples of stem cells include totipotent stem cells that have totipotency and are capable of differentiating into all the types of cells, and stem cells having pluripotency that can differentiate into the tridermal lineage as fetal stem cells can do, but having limited capability of differentiating into extraembryonic trophoblasts. Examples of stem cells further include multipotent stem cells that can differentiate into many cells of a tissue. Mesenchymal stem cells are known to undergo the transplanted site-specific differentiation. When mesenchymal stem cells are transplanted in the abdominal cavity of a sheep embryo, it is known that the cells differentiate into cartilage in cartilage tissue, skeletal muscle in skeletal muscle tissue, cardiac muscle in the heart, adipocytes in adipose tissue, and interstitial cells in the thymus or the bone marrow. Thus, mesenchymal stem cells are thought to be pluripotent. Because of such a property, it is considered that if a sufficient number of mesenchymal stem cells can be cultured, damaged tissues or organs can be regenerated by transplanting such mesenchymal stem cells so as to cause local differentiation of the cells. A preferred example of the mesenchymal stem cell is a stem cell that is obtained from interstitial cells attached to the bottom surface of a culture dish after primary culturing of bone marrow.

The precursor cells of mesenchymal cells refer to cells in the process of differentiation from mesenchymal stem cells to mesenchymal cells.

Mesenchymal cells differentiate from mesenchymal stem cells. Mesenchymal cells cannot undergo multidirectional differentiation as stem cells can do, but are capable of differentiating in a given direction and are capable of expanding. Under normal conditions, mesenchymal cells stay at phase G0, but can shift to phase G1 (initiation of division) when stimulated. Examples of mesenchymal cells include stromal cells and cells having the properties of stromal cells. Mesenchymal cells are present in every organ including subcutaneous tissue, lungs, liver, and mesenchymal tissue such as bone, cartilage, fat, tendon, skeletal muscle and the stroma of bone marrow.

Examples of cells derived from mesenchymal cells include (1) cells of the cardiovascular system such as endothelial cells or cardiac muscle cells or the precursor cells of the cells of the cardiovascular system, and cells having the properties of these cells; (2) cells of any one of bone, cartilage, tendon and skeletal muscle, the precursor cells of the cells of any one of bone, cartilage, tendon, skeletal muscle and adipose tissue, and the cells having the properties of these cells; (3) neural cells or the precursor cells of neural cells, and the cells having the properties of these cells; (4) endocrine cells or the precursor cells of endocrine cells, and the cells having the properties of these cells; (5) hematopoietic cells or the precursor cells of hematopoietic cells, and the cells having the properties of these cells; and (6) hepatocytes or the precursor cells of hepatocytes, and the cells having the properties of these cells.

Substances derived from immortalized cells refer to, for example, soluble cytokines and the like contained in a conditioned medium, adhesion molecules that are supplied by contact with cells and insoluble cytokine ligands. The expansion and differentiation of cells can be regulated by coculturing with these substances.

Examples of soluble cytokines include soluble SCF (kit ligand), flt3 ligand, thrombopoietin (TPO) and erythropoietin (EPO) in addition to insulin-like growth factor (IGF), various interleukins (interleukin-1, interleukin-2, interleukin-3, interleukin-12, interleukin-15, interleukin-18 and the like), various interferons, various factors to regulate expansion and differentiation (FGF, fibroblast growth factor; BDNF, brain derived neurotrophic factor; CNTF, ciliary neurotrophic factor; EGF, epidermal growth factor; G-CSF, granulocyte colony stimulating factor; GM-CSF, granulocyte/macrophage colony stimulating factor; M-CSF, macrophage colony stimulating factor; NGF, nerve growth factor; NT-3, Neurotrophin-3; NT-4, Neurotrophin-4; OSM, Oncostatin M; PDGF, platelet derived growth factor; TGFalpha, transforming growth factor alpha; TGFbeta, transforming growth factor beta; a secretory TNF family molecule group including a secretory CD40 ligand and the like, VEGF, vascular endothelial growth factor; Angiopoietin-1; Angiopoietin-2; PGF, placental growth factor/placenta-derived growth factor; and the like), and various chemokines (interleukin-8; RANTES; MIF, migration inhibitory factor; and MIP-1alpha and macrophage inflammatory protein-1 alpha).

Examples of so-called adhesion molecules and extracellular matrix acting on contact with cells include the integrin family, the cadherin family, the immunoglobulin superfamily, the selectin family, collagen (types I to XVI), fibronectin, elastin, the laminin group, osteocalcin, osteonectin, osteopontin, tenascin, thrombospondin, vitronectin and cartilage matrix protein.

Examples of insoluble cytokine ligands include a membrane-bound SCF (kit ligand), a membrane-bound CD40 ligand, and a membrane-bound TNF family molecule group containing TNF and the like.

Further, the present invention enables differentiation of mesenchymal stem cells into bone, cartilage and adipose tissue by immortalizing the cells and then employing appropriate conditions for inducing the differentiation of the cells. This is useful for intractable bone fracture and arthropathy. Stem cells are expanded by immortalizing the cells, so that it becomes possible to secure a number of the stem cells sufficient for treatment, and thus to use the cells practically.

The present invention further encompasses the construction of an artificial cell construct of cardiovascular system, artificial bone, artificial cartilage, artificial tendon, artificial skeletal muscle or artificial adipose tissue by coculturing the precursor cells of cardiovascular cells, bone, cartilage, skeletal muscle or adipose tissue with immortalized, mesenchymal system-related cells including mesenchymal stem cells, mesenchymal precursor cells, mesenchymal cells or cells derived from mesenchymal cells.

The present invention further encompasses the construction of a cell group that is part of the nervous system or a cell group coexisting in the nervous system, whose expansion or differentiation can be regulated, and the construction of a cell group that is part of the endocrine tissue or a cell group coexisting in the endocrine tissue, whose expansion or differentiation can be regulated by coculturing precursor cells of the nervous system or the same of the endocrine system with immortalized and mesenchymal system-related cells including mesenchymal stem cells, precursor cells of the mesenchymal cells and mesenchymal cells and cells derived from mesenchymal cells.

In addition, examples of the precursor cells of cardiovascular cells include the precursor cells of cardiac muscle derived from the heart tissue, myoblasts derived from skeletal muscle, vascular endothelial cell precursor cells in peripheral blood or bone marrow and angioblasts.

Examples of hematopoietic stem cells include a cell group containing hematopoietic stem cells in peripheral blood or bone marrow, and particularly CD34+ cells, a cell group containing hematopoietic stem cells of cord blood, and particularly CD 34+ cells, or hematopoietic stem cells derived from ES cells and CD 34+ cells.

Examples of "cells having the properties of the precursor cells of the cells of any one of bone, cartilage, tendon, skeletal muscle and adipose tissue" include osteoblasts, osteoclast-related cells, chondroblasts, myoblasts derived from skeletal muscle and adipose tissue-related cells.

Examples of "a cell group that is part of the nervous system or coexisting in the nervous system" include nerve stem cells, neurocytes, glial precursor cells, glia cells, and neurocytes forming retina and neural precursor cells.

Examples of "a cell group that is part of the endocrine tissue or a cell group coexisting in the endocrine tissue" include islets of Langerhans cells or the precursor cells thereof, and adrenal cells or the precursor cells thereof.

Further, the present invention also encompasses the fact that artificial bone marrow can be constructed by coculturing immortalized stromal cells with hematopoietic cells such as cord blood-derived CD34 positive cells.

### EXAMPLES

### [Example 1] Immortalization of stromal cells

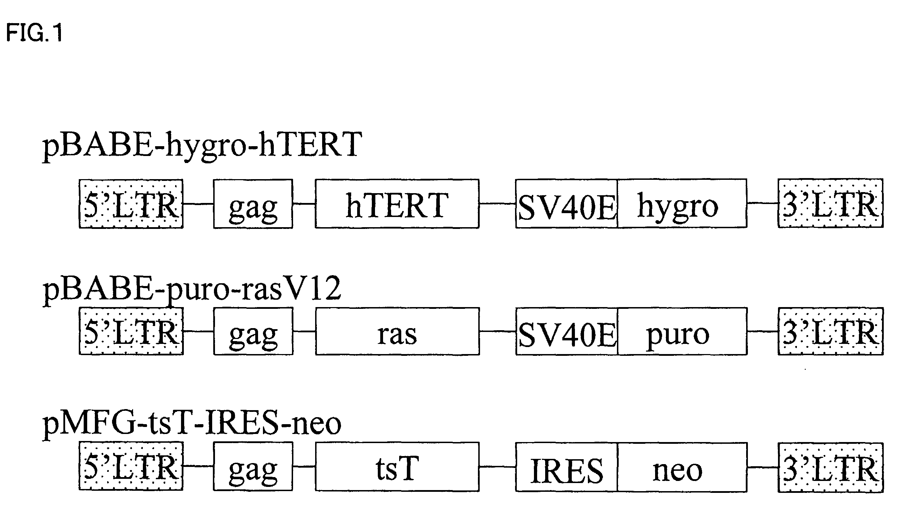

1. Collection of the primary stromal cells
   Mononuclear cells were isolated from 10 ml of bone marrow fluid that had been obtained from the ilium of a healthy adult man. After the mononuclear cells were cultured overnight, cells attached to a flask were used as stromal cells.
2. A gene encoding the catalytically active subunit (hTERT) of human telomerase was used as a gene to be transduced into a stromal cell. The sequence of hTERT is described in, for example, Science 277, pp. 955-959. Further, an ras gene and an SV40T gene that are known as genes relating to cell canceration were also transduced into stromal cells.
3. Vector to be used for transduction into stromal cells (Fig. 1)
   pBABE-hygro-hTERT (provided by Dr. Robert A Weinberg) was constructed by cloning an hTERT *Eco*R V-*Sal* I fragment, which had been obtained from pCI-Neo-hTERT-HA by PCR, into pBABE-hygro as described in Proc. Natl. Acad. Sci. USA vol 95, pp. 14723-14728. pBABE-puro-rasV12 (provided by Dr. Scott W Lowe) was constructed by the method described in Cell, 88, 593-602, 1997. pMFG-tsT-IRES-neo was constructed by cloning the BamH I fragment of IRES-neo [cleaved from pRx-hCD25-ires-neo (Human gene therapy 9, 1983-1993, 1998)] into MFG-tsT [obtained by cloning from pZIPtsU19 (provided by Dr. R. McKay) and incorporating it into a MFG vector (Lab. Invest. 78, 1467-1468, 1998)].
4. Preparation of retrovirus-producing cells and infection by the virus using the same were performed according to "A Separate Volume of Experimental Medicine, The Protocol Series, Experimental Protocols for Gene Introduction & Expression Analysis (ed., Izumi SAITOH and Sumio KANNO, YODOSHA, pp. 58-62)."

Specifically, using BOSC23 packaging cells (Proc. Natl. Acad. Sci. USA, 90: 8392-8396, 1993), ? CRIP packaging cells (Proc. Natl. Acad. Sci. USA, 90: 3539-3543, 1993) were prepared as described below.

### 4-1. Preparation of recombinant retrovirus vector-producing cells

(i) 5.5 x 10⁶ BOSC23 cells were inoculated on a 10 cm dish at 18 to 24 hours before transfection.
(ii) 800 µl of OPTI-MEM (Gibco/BRL) was added gently to 15 µg of DNA (retrovirus vector), and then agitated, thereby preparing solution A.
(iii) 750 µl of OPTI-MEM was collected in a sterilized tube. 50 µl of LIPOFECTAMINE (2 mg/ml Gibco/BRL) was added to the tube, and then slowly mixed, thereby preparing solution B.
(iv) Solution A was gently mixed with solution B to prepare solution C. Solution C was allowed to stand at room temperature for 30 to 45 minutes.
(v) BOSC23 cells were washed once with a medium at 37°C from which an antibiotic agent and FBS had been removed.
(vi) Solution C (1.6 ml) was added gently to BOSC23 cells.
(vii) 2.4 ml of OPTI-MEM was further added.
(viii) Incubation was performed for 5 hours under 5% CO₂.
(ix) 4 ml of DMEM containing 20% fetal calf serum was added, and then incubation was performed overnight.
(x) The medium was exchanged with a medium at 37°C containing 10% fetal calf serum, and 1 to 2 x 10⁶? CRIP packaging cells were inoculated on the 10 cm dish at the same time.
(xi) 24 hours later, the medium of BOSC23 cells was passed through a 0.45 or 0.20 µm syringe filter. The medium of ? CRIP was exchanged with a 5 ml of the filtered medium. At the same time, polybrene (Hexadimethrine Bromide, SIGMA H-9268) was added to 8 µg/ml.
(xii) After 4 to 24 hours of culture, 5 ml of a medium was added, followed by overnight culture.
(xiii) Drug selection was performed, so that the retrovirus-producing ? CRIP cells were prepared.

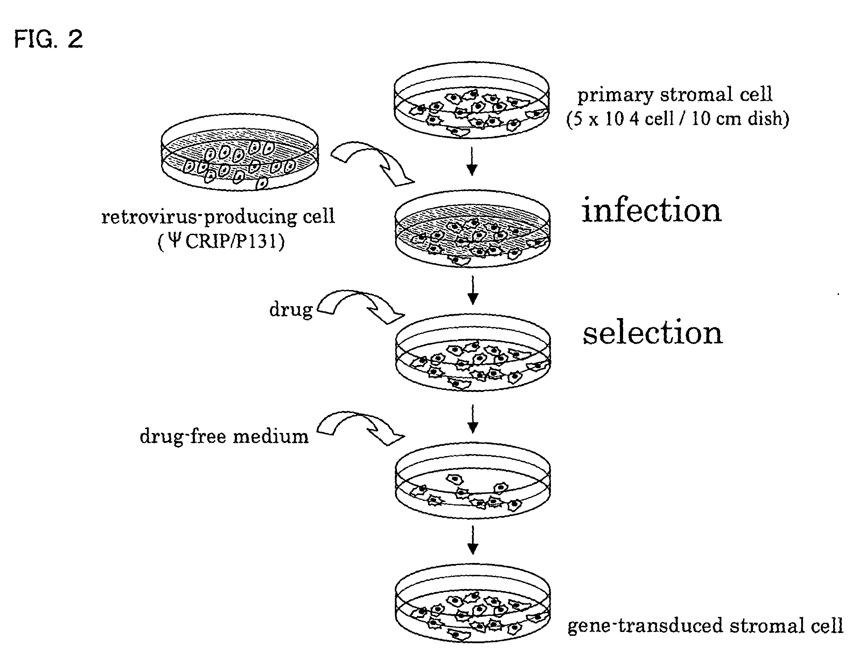

Next, the 3 above types of vectors were separately expanded by retrovirus-producing cells (? CRIP/P131), and then transduced (infection) into stromal cells as described below (Fig. 2).

First, on the day before infection, stromal cells were re-inoculated to a 5 x 10⁴ cell/10 cm dish, and then cultured after exchanging the medium of retrovirus-producing ? CRIP/P131, that is, 10% bovine serum-containing DMEM, with a 12.5% inactivated equine serum and 12.5% inactivated fetal calf serum/2-Mercaptoethanol/hydrocortisone-containing a-MEM medium. On that day, the culture supernatant was passed through a 0.20 µm filter, and then polybrene was added for a final concentration of 8 µg/ml. The recombinant retrovirus vector produced in the supernatant was then allowed to infect stromal cells. 4 hours later, the culture supernatant was exchanged with a new medium, followed by 2 days of culture. Subsequently, pBABE-hygro-hTERT was subjected to 5 days of drug selection using hygromycin (100 µg/ml), pBABE-puro-rasV12 was subjected to 5 days of drug selection using puromycin (1 µg/ml), and pMFG-tsT-IRES-neo was subjected to 5 days of drug selection using G418 (1 mg/ml).

Cells were infected using combinations of 3 types of retrovirus vectors; (1) control; (2) pBABE-hygro-hTERT vector only; (3) pMFG-tsT-IRES-neo vector only; (4) pBABE-puro-ras-V12 vector only; (5) 2 types of vectors, pMFG-tsT-IRES-neo and pBABE-hygro-hTERT; (6) 2 types of vectors, pBABE-puro-ras-V12 and pBABE-hygro-hTERT; (7) 2 types of vectors, pMFG-tsT-IRES-neo and pBABE-puro-ras-V12; and (8) 3 types of vectors, pBABE-puro-ras-V12 and pMFG-tsT-IRES-neo and pBABE-hygro-hTERT.

In addition, these viruses and cells are kept under conditions such that they are ready for subdivision at anytime after obtaining a patent.

When X-gal staining was performed for cells infected with pMFG-lacZ, staining was confirmed for approximately 3% of stromal cells.

Cell expansion after the drug selection was examined. Primary stromal cells (Primary) expanded for 43 days, and then ceased division with a generation number of 11 (PD (population doubling) = 11).

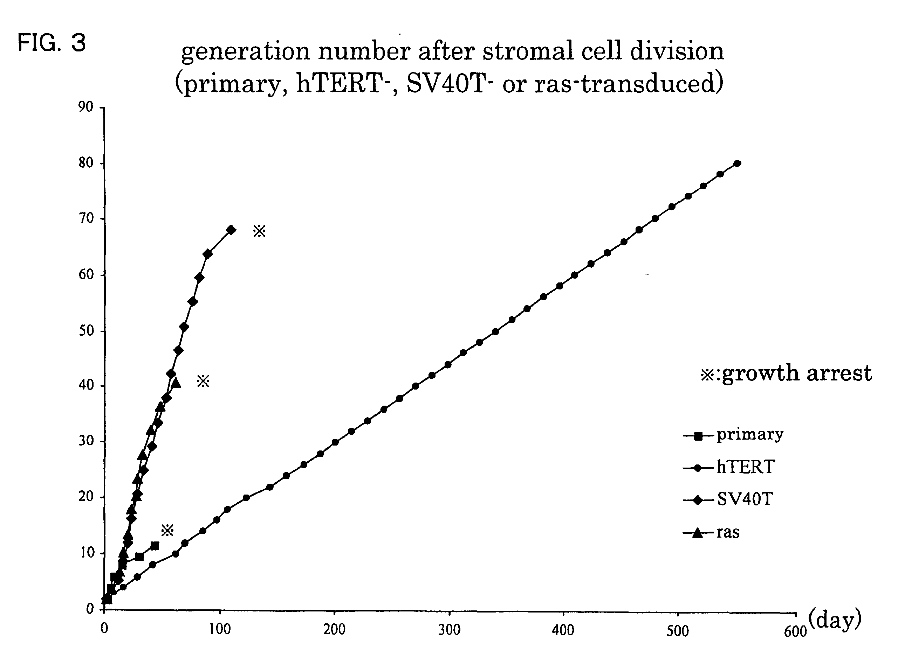

SV40T-transduced cells and ras-transduced cells both showed a higher division rate than that of the primary cells (primary), but ceased division on day 102 with a generation number of 67 (PD=67), and on day 61 with a generation number of 41 (PD=41), respectively. In contrast, hTERT-transduced cells were maintaining their division rate and can be subcultured, even now, after a lapse of 550 days and beyond a generation number of 80 (PD=80) (Fig. 3).

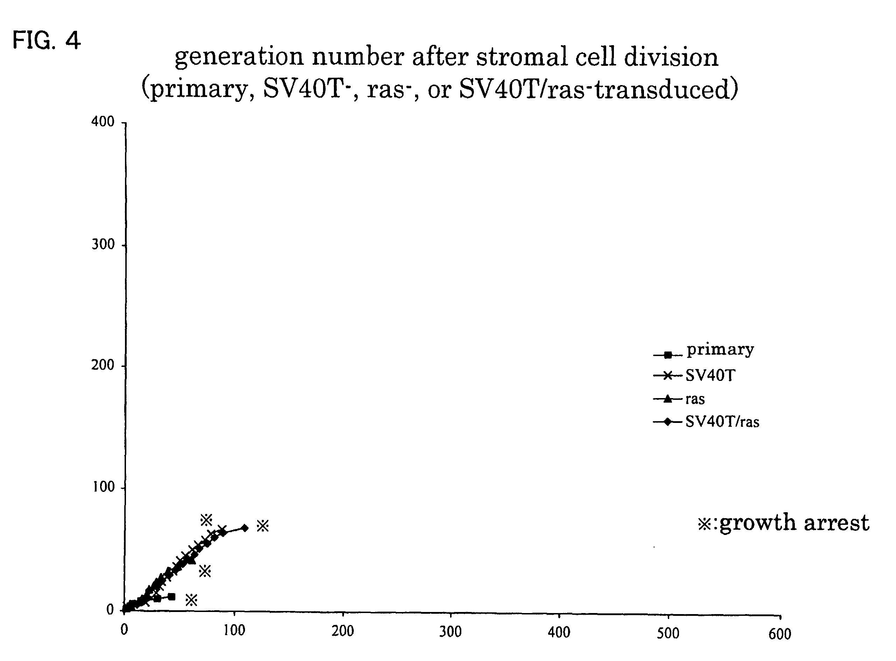

Next, mixed infection of SV40T and ras was studied. First, SV40T/ras ceased division on day 109 with a generation number of 68 (PD=68), which was almost the same behavior as that of the case of SV40T alone (Fig. 4).

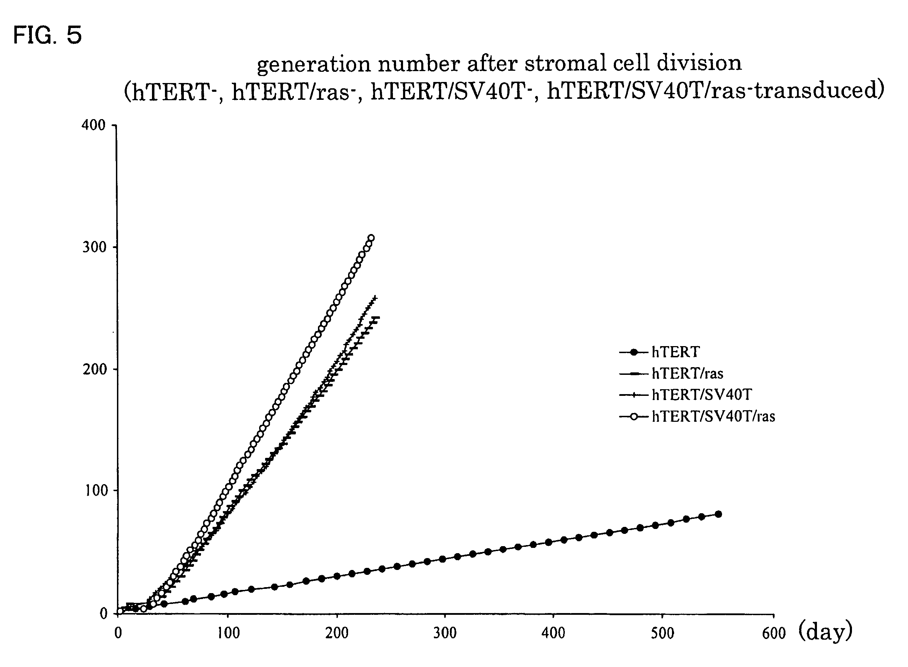

Further, SV40, ras and SV40T/ras, respectively having hTERT gene transduced therein, were studied (Fig. 5). hTERT/SV40T showed a generation number of 258 (PD=258) on day 235, hTERT/ras showed a generation number of 242 (PD=242) on day 236, and hTERT/SV40T/ras showed a generation number of 306 (PD=306) on day 232. These cells did not cease division at a rate which was the same as that of SV40T or ras alone, and could be subcultured. These cells were temporarily cryopreserved. When thawed and cultured again, these cells can be similarly subcultured. According to the above results, hTERT alone or hTERT/SV40T, hTERT/ras and hTERT/SV40T/ras (all of the three cases contained hTERT) could be subcultured with generation numbers far greater than 70 (PD=70) or more on day 100 at which ras or SV40 alone or SV40T/ras ceased division. Therefore, we defined these cells as immortalized.

Cases other than that wherein hTERT gene was transduced alone were all contact inhibition-free, and in particular, ras-transduced stromal cells showed expansion also in a vertical direction.

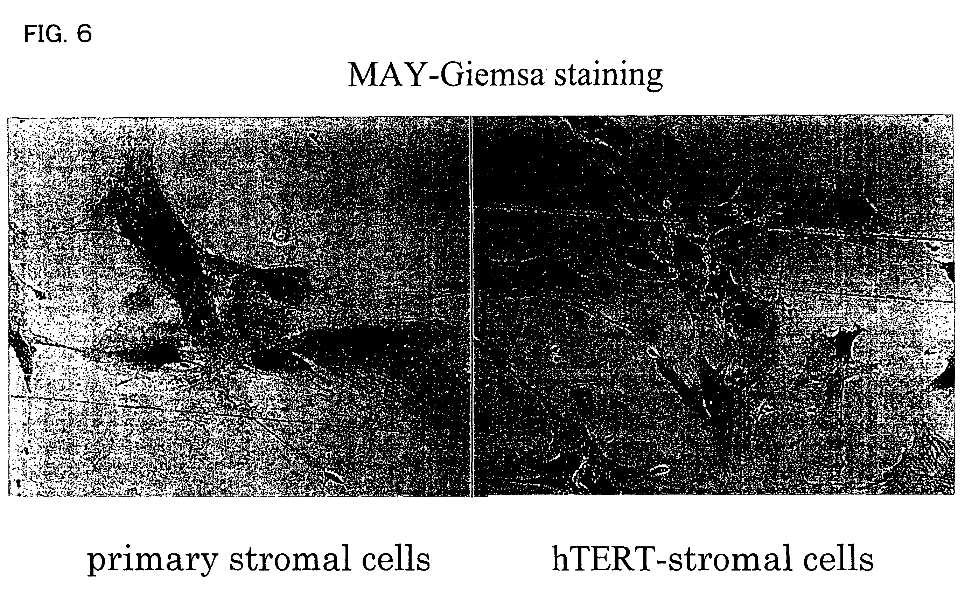

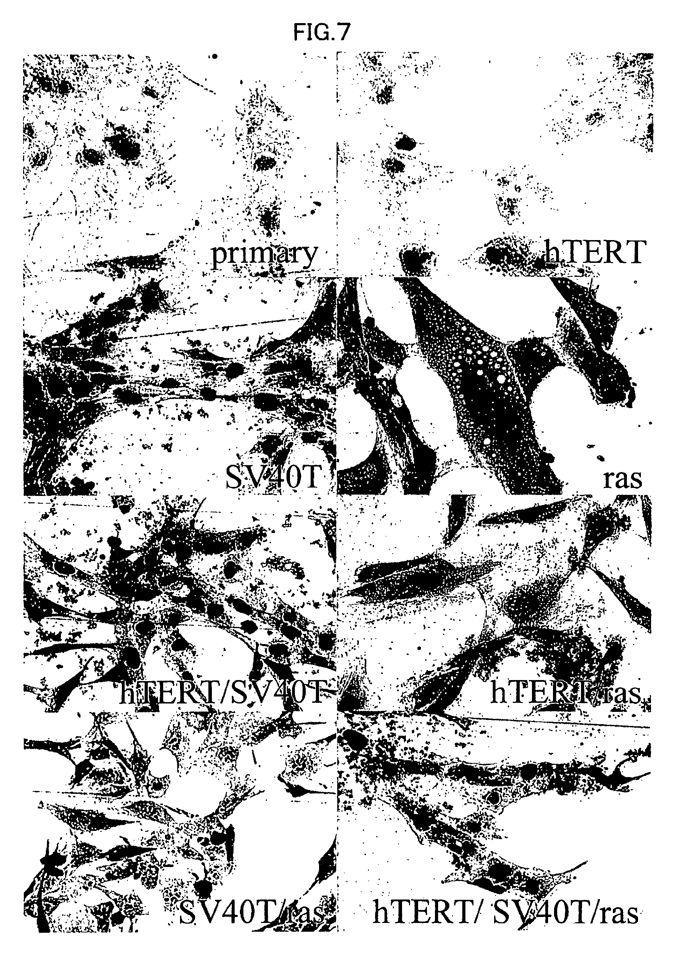

To morphologically observe these stromal cells, May-Giemsa staining was performed (Fig. 6). As a result, the form of hTERT gene alone-transduced stromal cells was the closest to that of the control (Fig. 7). The size of SV40T gene-transduced cells, and those of SV40T gene and hTERT-transduced cells were somewhat smaller than that of the control. The ras gene-transduced stromal cells formed vacuoles in their cytoplasms.

### [Example 2] Study of telomerase activity

To confirm the expression of hTERT gene transduced into a target cell and the generation of hTERT activity, telomerase activity was examined using a Telo Chaser of TOYOBO.

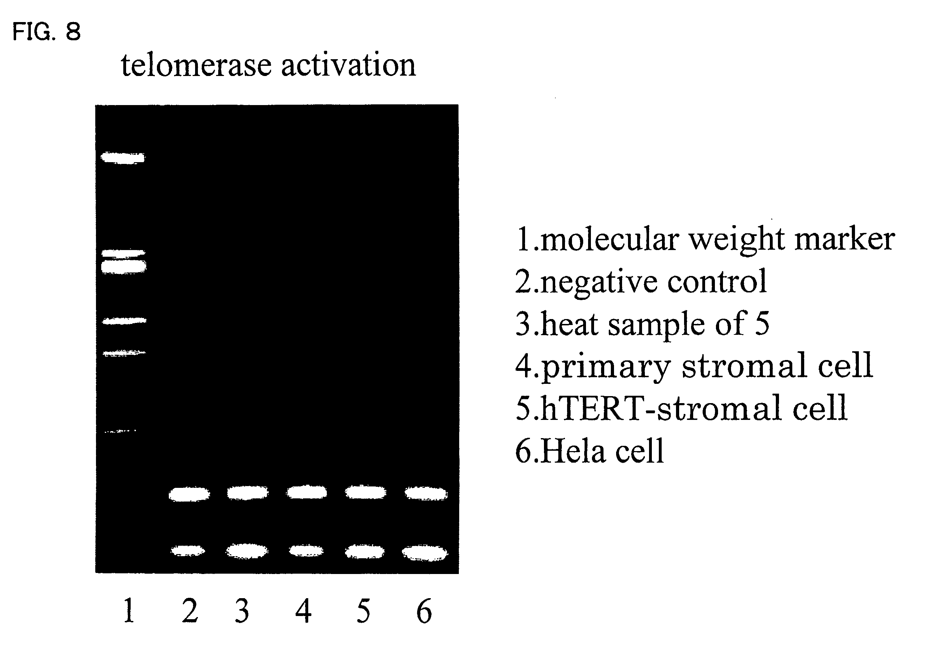

Telomerase activity was measured according to the protocols of Telo Chaser (TOYOBO) using Hela samples attached to the kit as a positive control. Telomerase was extracted respectively from stromal cells, hTERT gene-transduced stromal cells, and Hela cells that had been isolated by a method similar to that of Example 1. Using telomerase extracted from each type of cells, and telomerase heat-treated at 70°C for 10 minutes after extraction from hTERT gene-transduced stromal cells, a reaction to add telomeric repeats to a substrate primer was performed, PCR was performed using reverse primers, and then polyacrylamide gel electrophoresis was performed for visualization. Figure 8 shows the results. In Fig. 8, 1 indicates the molecular weight marker, 2 indicates the cytolytic solution only (negative control), 3 indicates the sample of hTERT gene-transduced stromal cells heat-treated at 70°C for 10 minutes, 4 indicates primary culture stromal cells, 5 indicates hTERT gene-transduced stromal cells, and 6 indicates Hela cells that are oncocytes (Hela cell positive control). As shown in Fig. 8, it could be confirmed that hTERT gene-transduced stromal cells had hTERT activity, because the band of 5 is at the same level of that of 6. Specifically, it was confirmed that the transduced gene expressed hTERT, and thus hTERT activity was present.

Further, telomere length was measured using a Telo TAGGG telomere length assay (Roche Molecular Biochemicals, Sandhofer, Germany). In sum, the genomic DNA was denatured using restriction enzymes, Rsa I and Hinf I, and then isolated by 0.8% agarose gel. DNA was transferred to a nylon membrane using a capillary. Hybridization was performed using a telomere-specific probe labeled with digoxigenin.

Telomerase activity was then measured, and then the telomere lengths of hTERT-stromal cells were measured for a long period (Fig. 33). Telomerase activity was detected at PD=10, 60 and 100. The average telomere length was between 6 and 20 kb and was maintained during the period, suggesting that the enhanced telomerase activity contributed to the maintenance of telomere length.

### [Example 3] Characterization of stromal cell line

### (1) Study of surface antigen

Bone-marrow fluid was collected from the ilia of NK and KY of healthy individuals by bone marrow aspiration, and then mononuclear cells were separated by densimetric centrifugation. The obtained cells were cultured overnight, and then on the next day the cells attached to the flask were used as primary stromal cells.

Similar to Example 1, hTERT gene was transduced into a stromal cell using pBABE-hygro-hTERT.

Primary stromal cells and hTERT gene-transduced stromal cells were subjected to the expression analysis of cell surface antigens using FACS. The antibodies used herein were: CD45, CD9, CD 105 (SH2), CD73 (SH3), CD 166 (ALCAM) and CD 157 (BST-1). Data collection and analysis were performed using Cell Quest. CD45 and CD9 were obtained from Immunotech, Marseille, France; CD166 (ALCAM) was obtained from Antigenix America, Huntington, USA; CD105 was obtained from Ancell, Bayport, USA; CD73 (SH-2) was obtained from Alexis biochemicals; and CD157 (BST-1) was obtained from MBL, Nagoya, Japan.

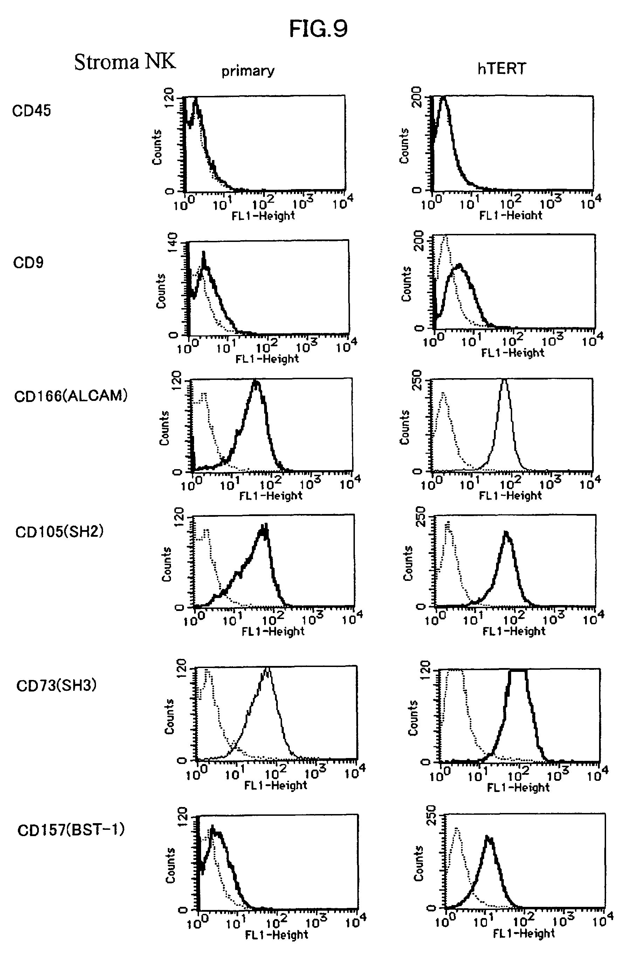

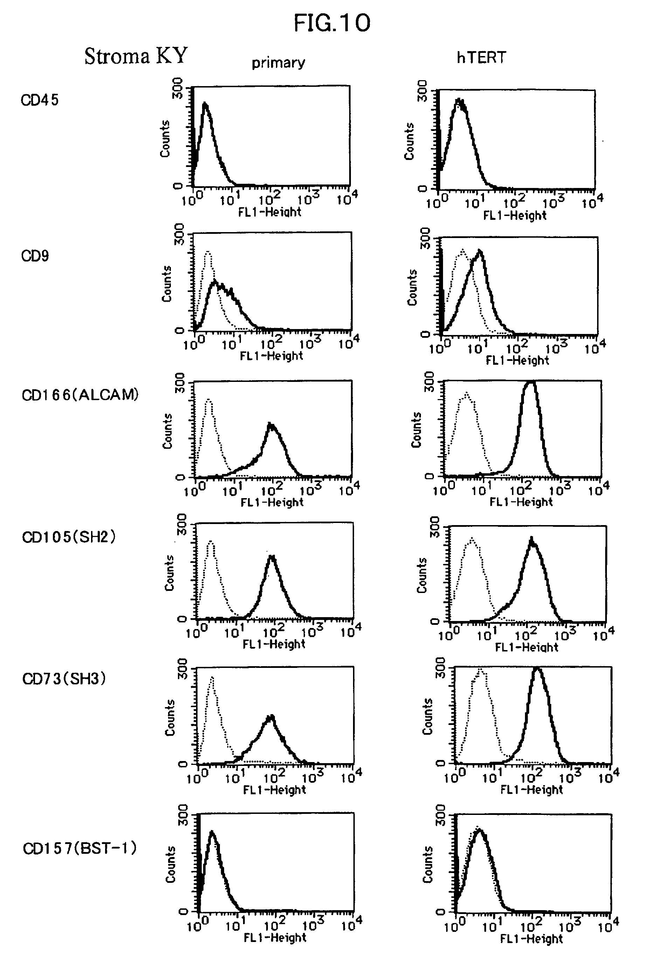

As shown in Figs 9 and 10, the results of this analysis for all of the 4 types of cells were: CD45(-), CD9(+), CD166 (ALCAM) (+), CD105(SH2)(+) and CD73(SH3)(+). Stroma NK showed (+) against CD157 (BST-1), and Stroma KY showed (-) against CD157 (BST-1), while no change was observed for the expression of CD157 (BST-1) between primary stromal cells and hTERT gene-transduced stromal cells.

### (2) Chromosome type

When karyotyping of hTERT gene-transduced stromal cells (used in (1) above) was performed, the chromosome number was 46 (normal), and neither deletion nor translocation of chromosomes was observed.

### (3) Expression of cytokine

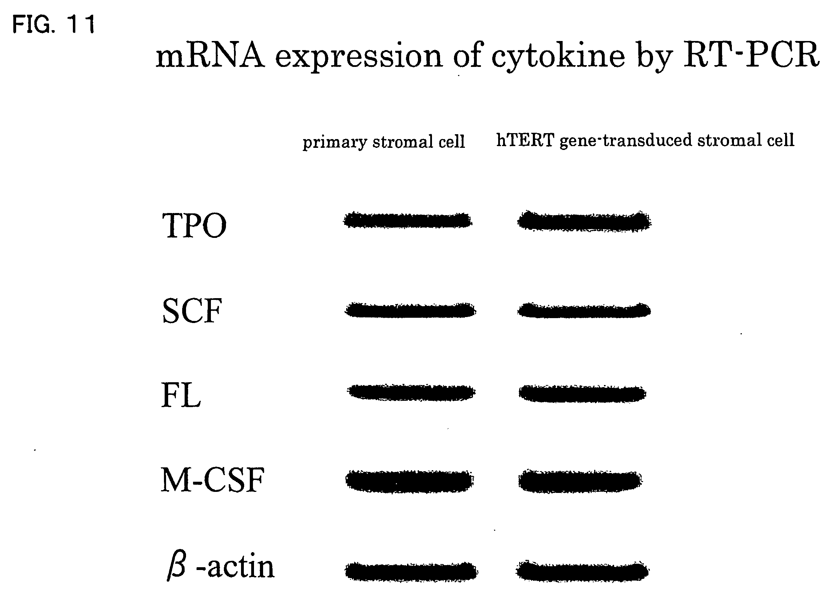

The expression of cytokines produced from stromal cells was studied at the mRNA level. RNA was extracted from the cells, and then cDNA was prepared using reverse transcriptase. PCR was performed for the cytokines TPO, SCF, FL and M-SCF using the cDNA as a template and primers corresponding to the cytokines. Band amplification was confirmed by agarose gel electrphoresis. The expression of the 4 above types of cytokines was observed for both types of the cells (Fig. 11).

### [Example 4] Coculture of CD34 (+) cord blood cells and stromal cell line

The supporting capacity of the blood stem cells of stromal cells that had been subjected to gene introduction similar to Example 1 was first verified by colony assay. Gene introduction was performed using the same vector as used in Example 1 under the conditions of (2), (3), (5), (6), (7) and (8) for the infection with retrovirus vectors in Example 1. Specifically, the cells (primary stromal cells) stored at the time of bone marrow collection were used as control, and the cells were infected with retroviruses, followed by the drug selection. The thus obtained stromal cells having 1 to 3 types of genes transduced therein were cultured for 3 months.

The stromal cells infected with retroviruses were inoculated again in a 25 cm² flask. When the cells reached subconfluence, they were irradiated with 21 to 22 Gy to arrest cell growth. Then the medium of the stromal cells was removed. Next, removal was repeated after the addition of X-VIVO10, so that serum contained in the stromal medium was removed. Then X-VIVO 10 supplemented with TPO (50 ng/ml), FL (50 ng/ml) and SCF (10 ng/ml) were added, and then 5 x 10³ CD34+ cord blood cells that had been previously put in X-VIVO 10 (with TPO (50 ng/ml), FL (50 ng/ml) and SCF (10 ng/ml)) was added. Thus, in the presence of CD34+ cord blood cells, coculture was performed for 2 weeks with the stromal cells that had been subjected to transduction using the vector. The culture supernatant was collected, and then 2 × 10³ cells were used for colony assay. 2 weeks later, colonies were counted. The results are shown in Table 1 below.

Among the cocultured stromal cells, colony assay could be performed only for the control (primary stromal cells) and stromal cells having only the hTERT gene transduced therein. Culturing of the remainder could not be continued because the stromal cells came off the culture plates during the coculture. The cell death may be caused by apoptosis due to radiation exposure or failure in suppression of growth. Cicuttini et al. reported that the expansion of SV40 T gene-transduced stromal cells could not be regulated by radiation exposure (Blood, 80: 1992, 102-112).

**Table 1**

| Number of colonies formed | Primary stromal cells | hTERT gene-transduced stromal cells |
|---|---|---|
| Before coculture | 3300 | 3300 |
| After coculture | 73500 | 48900 |
| Amplification rate | 22.3 | 14.8 |

The amplification rates of the number of colonies in the cases of control and transduction of the hTERT gene alone were 22.3-fold and 14.8-fold, respectively. From the results, it was confirmed that hTERT possesses to some degree a capacity for supporting blood stem cells even after 3 months of culture, and even the established stromal cells can be cocultured.

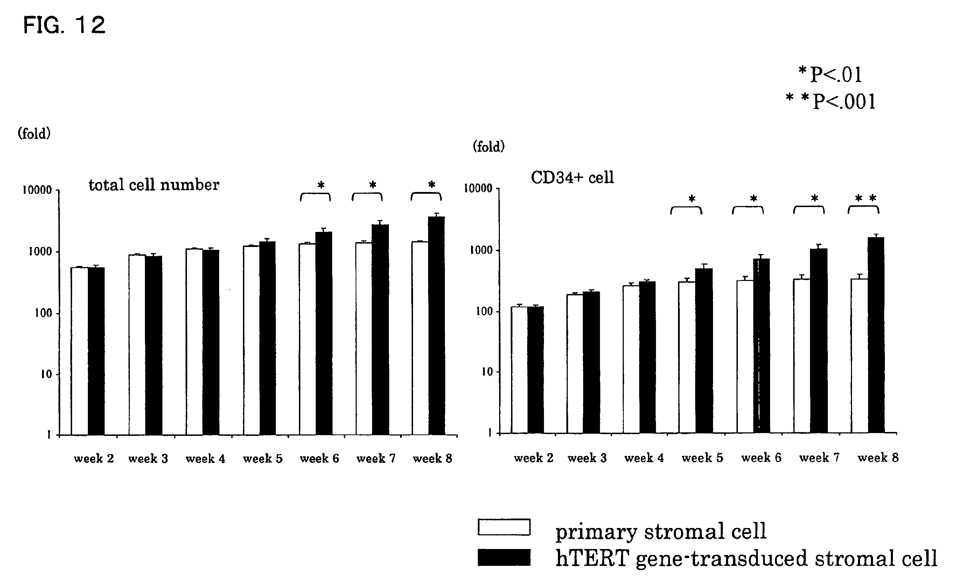

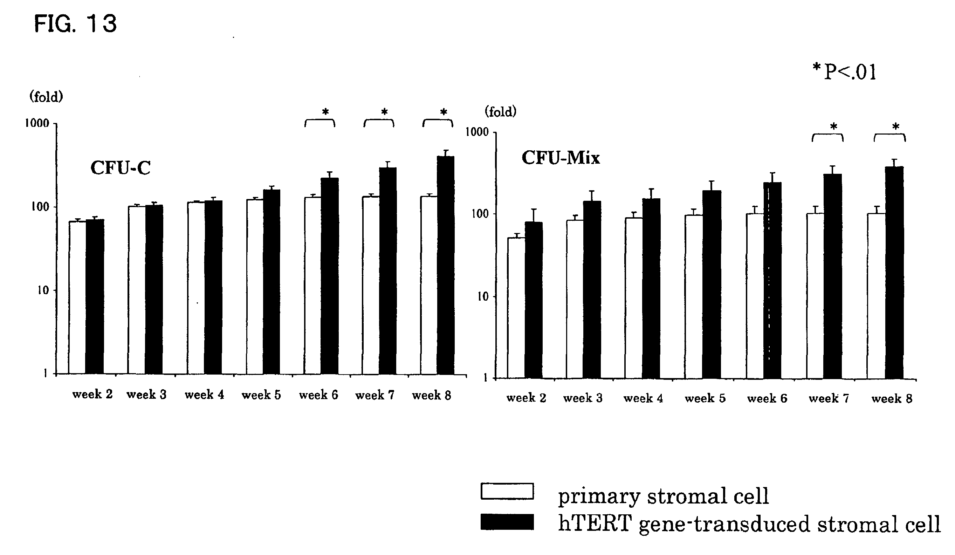

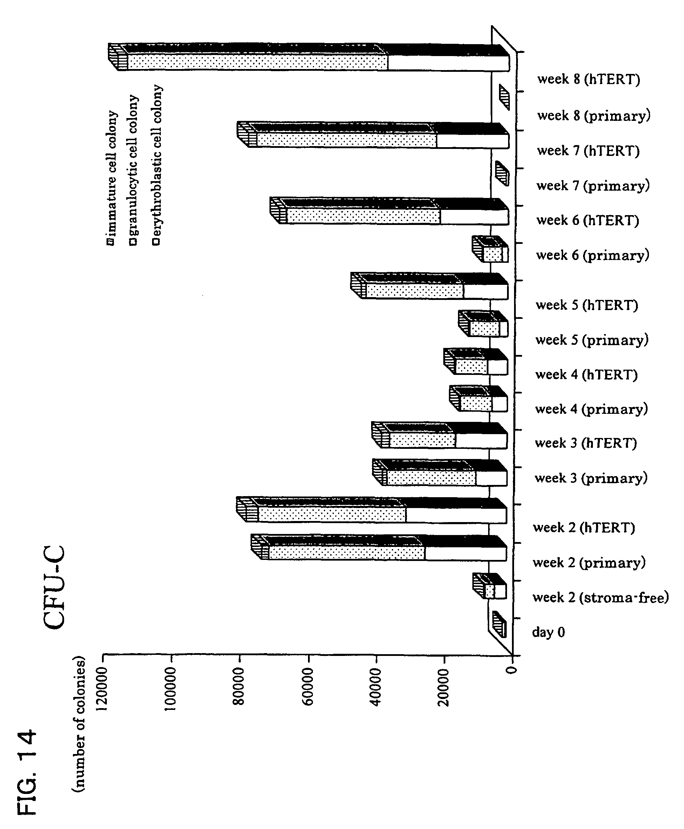

Next, to confirm reproducibility, 5×10³ CD34+ cord blood cells were added, and then cultured using a serum-free medium of X-VIVO 10 supplemented with 50 ng/ml TPO, 50 ng/ml FL and 10 ng/ml SCF. On day 7, the same medium was added again, and then coculture was performed again. From week 2, only the suspended cells were collected (cells that had gotten into the stromal cells were not collected). After that time, cells were collected every week, and a study was conducted as described above until week 8 concerning total cell number, number of CD34+ cells, number of colonies formed (CFU-C), number of immature colonies (CFU-Mix), granulocytic cell colonies and erythroblastic cell colonies. The results are shown in Figs. 12 to 14.

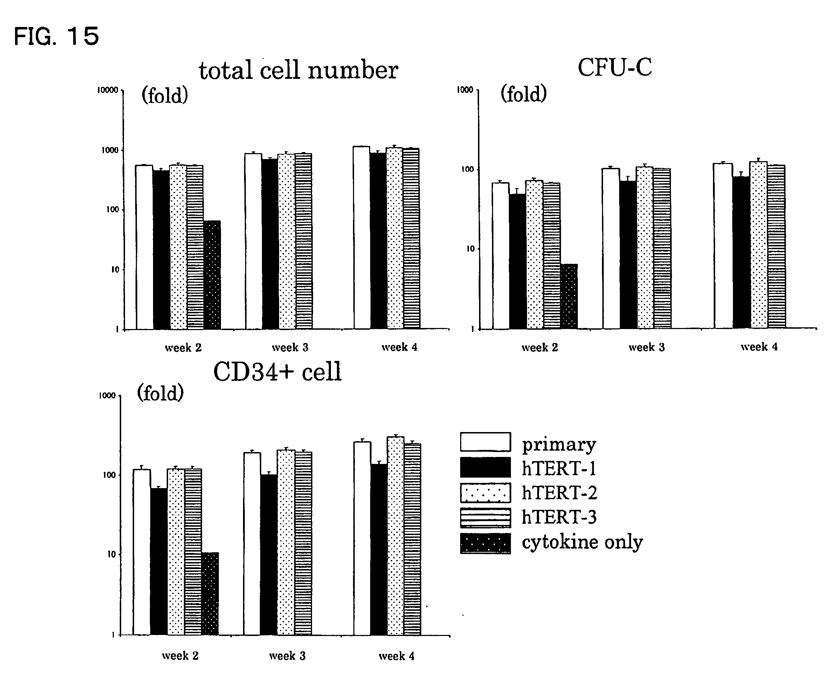

Furthermore, comparison was made among a case involving the absence of stromal cells, and cases involving stromal cell hTERT 2 and hTERT3 that had been established by transducing hTERT gene into stromal cells collected from other healthy individuals. The results are shown in Fig. 15. For the case involving the absence of stromal cells, blood cells had already died on day 14, so that assay was impossible. When compared with the primary stromal cells, equivalent expansions were shown until day 28, but after that day, hTERT gene-transduced stromal cells alone maintained expansion potency.

Each stromal cell having hTERT-transduced therein showed slight differences depending on the samples, but showed, until day 35, in vitro expansion in total cell number, CD34+ cell and CFU-C.

### [Example 5] Differentiation potency of mesenchymal stem cells

Next, differentiation of mesenchymal stem cells having capability of both differentiation into bone, cartilage, muscle and the like, and autoreproduction was studied, as was the supporting capacity of blood stem cells.

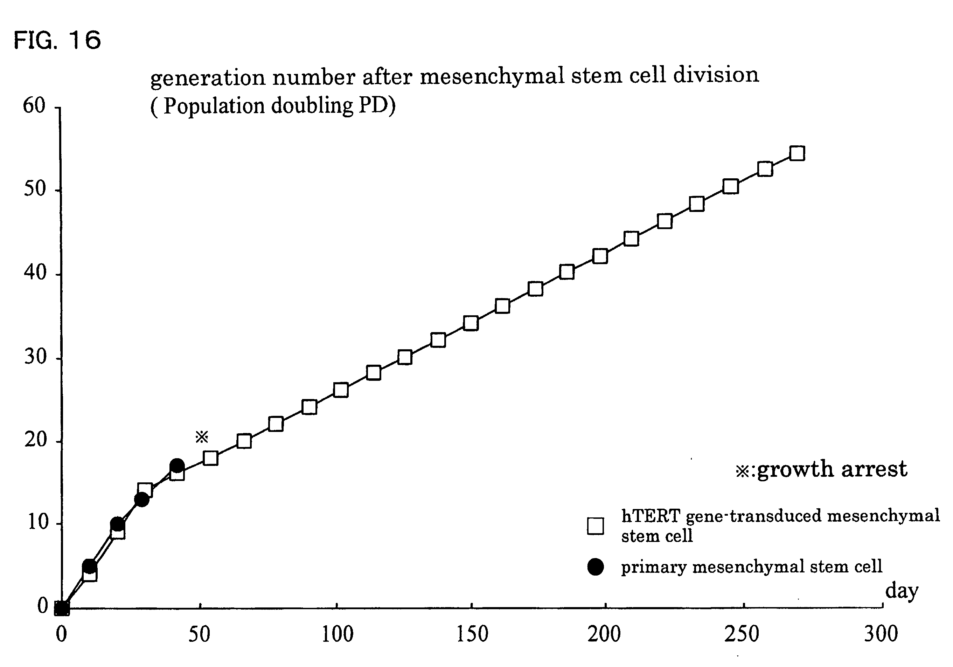

Bone marrow aspiration was performed for the ilia of healthy individuals, and then mononuclear cells were collected by densimetric centrifugation. The obtained cells were cultured overnight in a 10% inactivated fetal calf serum-containing DMEM. From the next day, the adherent cells were cultured. 2 weeks later, the cells collected using T-E (trypsin-EDTA) were cryopreserved as primary mesenchymal stem cells. Subsequently, the hTERT gene was transduced into the cells similar to Example 1. Next, growth curve was compared between the primary mesenchymal stem cells and hTERT gene-transduced immortalized mesenchymal stem cells. The results are shown in Fig. 16. As clearly indicated in Fig. 16, although the primary mesenchymal stem cells ceased cell division (crisis) with a generation number of 17 (PD=17) on day 42, the immortalized mesenchymal stem cells can be subcultured without decreasing their division rate even now, beyond a generation number of 54 (PD=54) on day 270. Thus, it was considered that a stable cell line was established.

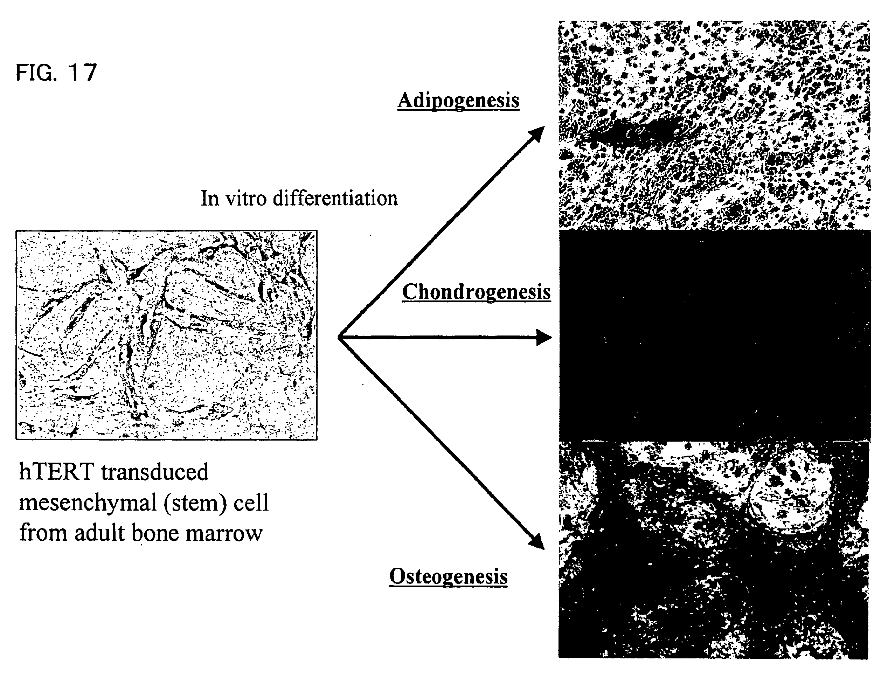

Next, it was studied whether the prepared mesenchymal stem cells possess pluripotency.
(1) First, differentiation into adipocytes (adipogenesis) was induced using 1 µM dexamethazone, 60 µM indomethacine, 0.5 µM 3-isobutyl-1-methylxanthine (isobutylmethylxanthine) and 5 µg/ml insulin. After approximately 1 week of culture, the cells were stained with Oil Red 0 stain (the fatty drop is red).
(2) Subsequently, cartilage differentiation (chondrogenesis) was induced using 1 µM dexamethazone, 50 µg/ml ascorbate-2-phosphate, 6.25 µg/ml insulin, 6.25 µg/ml transferrin, 5.35 µg/ml selenic acid, 1.25 mg/ml linoleic acid and 10 ng/ml TGP-β. After 2 to 3 weeks of culture, the cells were stained with Alcian blue. Chondroitin within the stained (frozen sections) cartilage matrix was stained blue.
(3) Furthermore, bone differentiation (osteogenesis) was induced using 1 µM dexamethazone, 50 µM ascorbate-2-phosphate and 10 mM β-glycerophosphate. After 2 to 3 weeks of culture, the cells were stained with von Kossa stain (mineral deposition).
The results are shown in Fig. 17.

### [Example 6] Differentiation from immortalized mesenchymal stem cells to immortalized supporting cells that support blood cells

Supporting capacity for blood cells was studied by exchanging a medium of mesenchymal stem cells that had been collected and immortalized by a method similar to that of Example 5 with a medium for supporting cells.

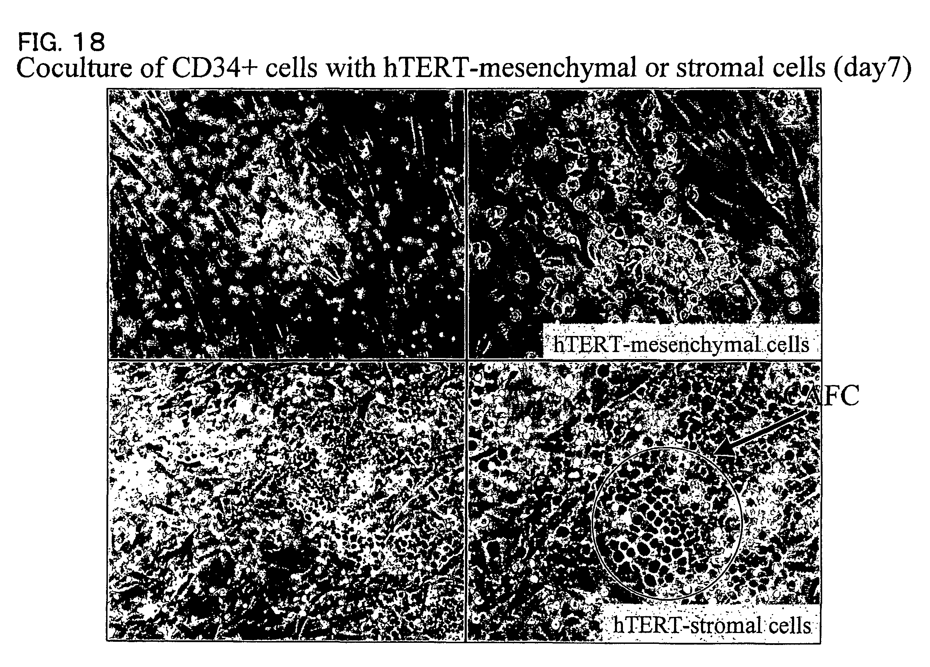

After the medium of the mesenchymal stem cells (10% inactivated fetal calf serum-containing DMEM) was exchanged with the medium of supporting cells (stromal cells) (composition: 12.5% inactivated fetal calf serum, 12.5% inactivated equine serum, 1×10⁻⁶ M hydrocortisone and 10⁻⁴ M 2-ME-containing a-MEM medium), coculture with cord blood CD34 positive cells was performed. On day 7 after the coculture, macroscopic observation was performed. In Fig. 18, coculture with immortalized mesenchymal stem cells is shown at the top, and coculture with immortalized supporting cells is shown at the bottom. The pictures on the left were obtained by low power magnification, and those on the right were obtained by high power magnification. Amplification of the blood cells was observed in all the cases (no difference in number was found between the two on day 7). In the case of coculture with immortalized supporting cells, cobblestone area-forming cells (CAFC) that were thought to be a population of immature blood cells were observed. This may have been caused by the fact that mesenchymal stem cells sufficiently differentiated into supporting cells, even though the mesenchymal stem cells had been immortalized.

### [Example 7] Production of erythroid precursor cells

Stromal cells that had been immortalized by transducing hTERT gene in the manner similar to Example 1 were inoculated in a 25 cm² flask. When the cells reached subconfluence, the cells were subjected to 21 to 22 Gy X-radiation so as to stop cell expansion.

5×10³ CD34+cord blood cells were cocultured with stromal cells in the presence of TPO (50 ng/ml), FL (50 ng/ml) and SCF (10 ng/ml) for 2 weeks or 4 weeks, so that hematopoietic precursor·stem cells were amplified (stem cell amplification phase). Hemocytes amplified on the stroma were collected, the number of the cells was counted and the proportion of Glycophorin A (GPA) positive cells was analyzed by flow cytometry.

The total number of cells collected on day 14 of the stem cell amplification phase was 2×10⁶ . The cells could be amplified to a number approximately 400-fold greater than the cell number of 5000 (before amplification). Among these cells, the proportion and the number of GPA positive cells, the markers for erythroblasts, were 22.9% and 2.9×10⁴, respectively. On day 28 of the stem cell amplification phase, the yield was as high as the total cell number of 3.6×10⁶ , and the proportion and the number of GPA positive cells were 2.0% and 6.1×10³, respectively.

The amplified 5×10³ hematopoietic precursor stem cells were cultured for 8 days in a medium for inducing differentiation: (1) Erythropoietin (EPO) induction medium (composition: X-VIVO 10, 500 µg/ml diferric transferrin, 1 % deionized bovine serum albumin (BSA) and 2 U/ml EPO) or (2) Erythropoietin in combination with stem cell factor (SCF) medium (composition: X-VIVO 10, 500 µg/ml diferric transferrin, 1 % deionized bovine serum albumin (BSA), 2 U/ml EPO and SCF 10 ng/ml) so as to induce erythroblast production (erythroblast induction phase).

The thus obtained erythroblasts were analyzed by flow cytometry involving photomicroscopic images of the inside of the culture dish, smears, total cell number and GPA positive cell proportion.

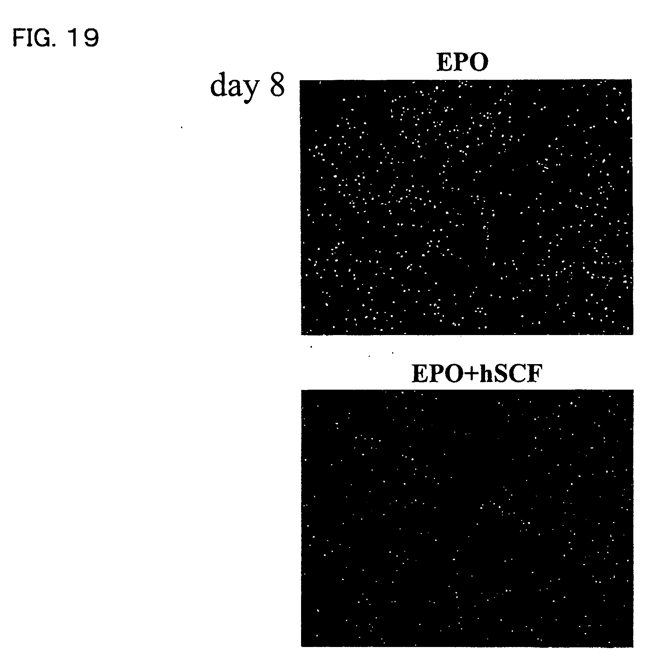

Fig. 19 shows findings about cultured erythroblasts on day 8 of the erythroblast induction phase. Pictures at the top and at the bottom show the results of inducing the differentiation of erythroblasts using an Erythropoietin (EPO) medium, and a medium containing both EPO and stem cell factor (SCF), respectively. When the differentiation of erythroblasts was induced by EPO alone, the total cell number and the number of clusters formed were very low. In contrast, when differentiation was induced by the combined use of SCF and EPO, the total cell number and the number of clusters formed were shown to be extremely abundant.

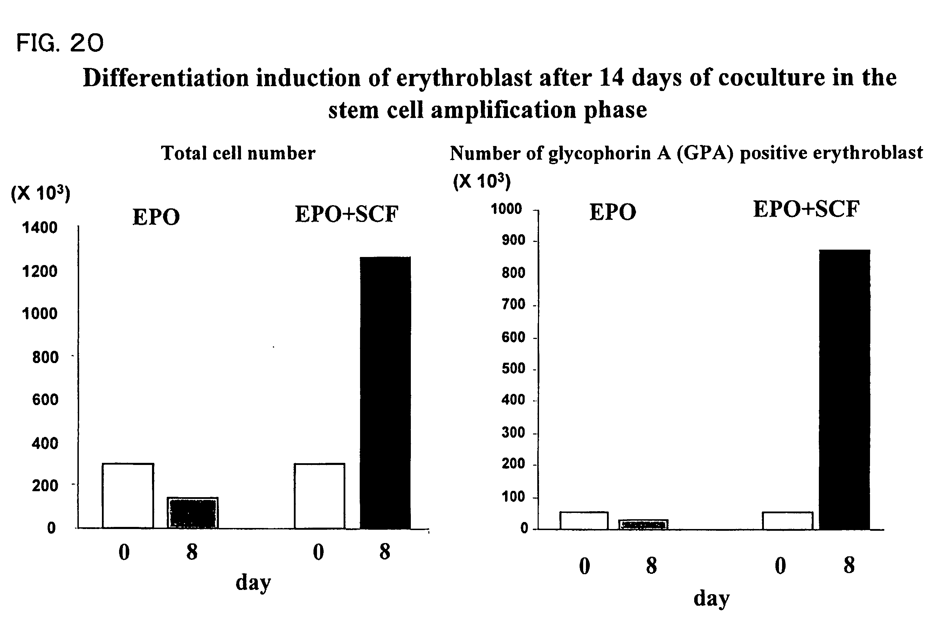

Fig. 20 shows the results of promoting erythroblast production using two types of media for inducing differentiation on day 14 of the stem cell amplification phase. In this figure, total cell number is shown on the left and the number of GPA positive erythroblasts is shown on the right. In the figure, EPO indicates the results of inducing differentiation using an EPO medium, and EPO+SCF indicates the results of inducing differentiation using a medium containing both EPO and SCF. When differentiation was induced by EPO alone, which is a conventional method, a decrease from 3×10⁵ (before amplification) to 1.4×10⁵ was observed in total cell number. When SCF was used in combination with EPO, the total cell number was amplified to 1.3×10⁶, which was approximately 4.2-fold greater than the number before amplification. Further, the number of GPA positive erythroblasts was observed to decrease from 5.3×10⁴ (before amplification) to 2.9×10⁴ when induced by EPO alone, while the same was observed to amplify to 8.7×10⁵, which was approximately 16.4-fold greater than the number before amplification, when SCF was used in combination with EPO.

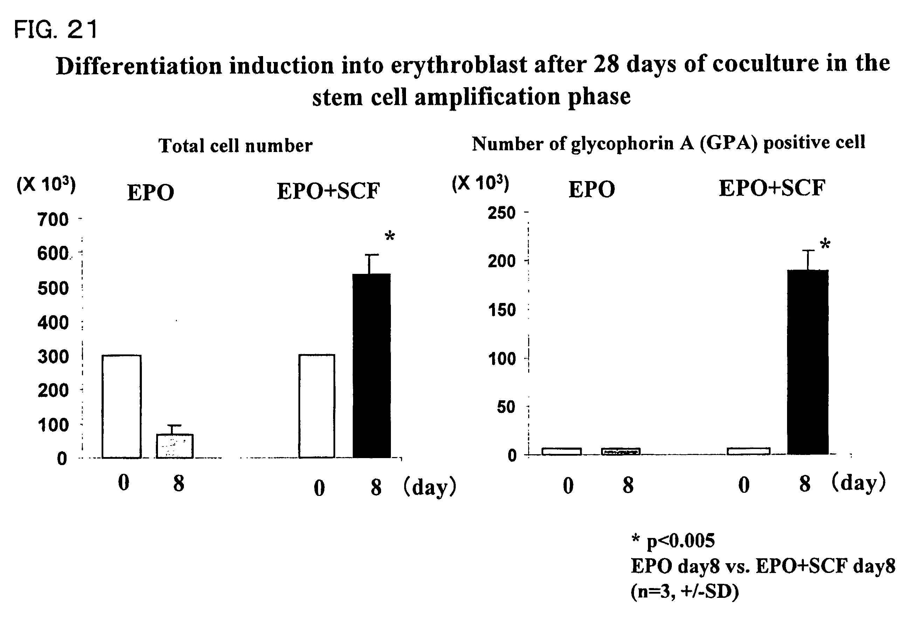

Figure 21 shows the results of inducing erythroblast production on day 28 of the stem cell amplification phase. Similar to the results of Fig. 20, total cell number was shown to decrease from 3x10⁵ (before amplification) to 6.7x10⁴ in the case of EPO alone. However, in the case of using SCF with EPO, total cell number was observed to amplify to 5.3x10⁵, which was approximately 1.7-fold greater than 3x10⁵ (before amplification). Further, GPA positive erythroblasts were shown to decrease in number in the case of EPO alone, while in the case of using SCF with EPO, the cell number was observed to amplify to 1.9x10⁵, which was approximately 31-fold greater than 6.1x10³ (before amplification).

### Findings of erythroblast by May-Giemsa staining

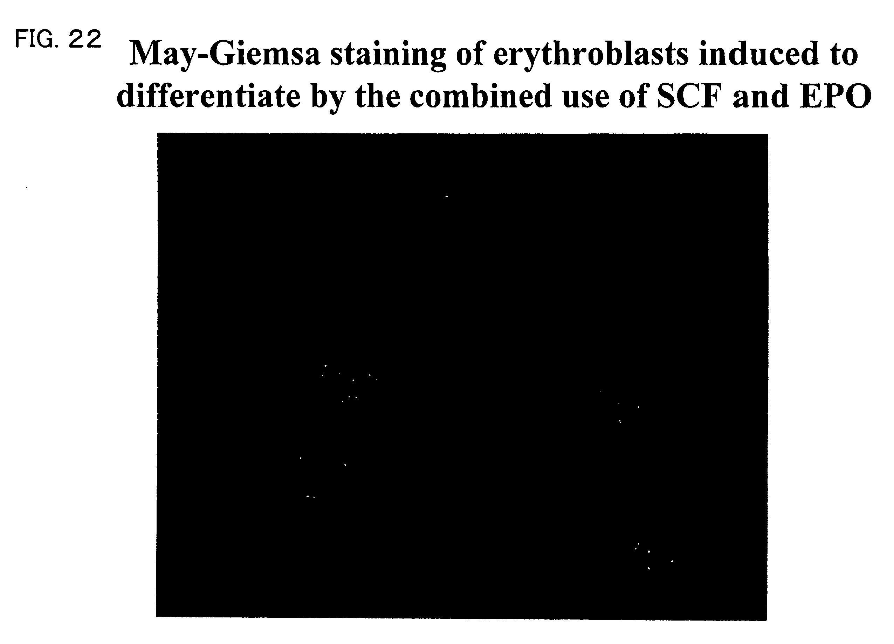

Figure 22 shows the findings by May-Giemsa staining on day 8 after differentiation induction of erythroblasts using SCF with EPO. It was shown that almost all the hemocytes were juvenile erythroblasts having basophilic cytoplasm and nuclei that were round and roughly produced. That is, it was considered that not only GPA positive cells but also GPA negative cells are erythroblasts, and they are very juvenile, to the extent that they do not mature enough to express GPA.

Further erythroblast induction using erythrpoietin alone or using both erythropoietin and SCF is described in the following references:
1. Shintani N, Niitsu Y. et al. Expression and extracellular release of transferrin receptors during peripheral erythroid progenitor cell differentiation in liquid culture. Blood, 83. 1994:1209-1215.
2. Kapur R., Zhang L. et al. A novel mechanism of cooperation between c-Kit and erythropoietin receptor. Stem cell factor induces the expression of Stat5 and erythropoietin receptor, resulting in efficient proliferation and survival by erythropoietin. J Biol Chem 276 2001: 1099-1106.

### [Example 8] Differentiation/expansion of cord blood-derived CD34 positive cells into mature erythrocytes using immortalized stromal cells

### (1) Method:

CD34 positive cells were separated from cord blood using a MACS separation kit (Miltenyi Biotec). Differentiation and expansion into erythrocytes involved 3 steps. In the 1st phase (days 0 to 14), CD34 positive cells were suspended at a concentration of 5x10³/3 ml together with immortalized stromal cells that had reached confluence in a medium of X-VIVO10 supplemented with Stem Cell Factor (SCF) 10 ng/ml, Thrombopoietin (TPO) 50 ng/ml and Flt-3/Flk-2 Ligand (FL) 50 ng/ml in a 25 cm² flask. Then the cells were cultured for 14 days. On day 7, 3 ml of a medium with a composition the same as that described above was added. In the 2nd phase (days 14 to 28), the cells were suspended at a concentration of 1x10⁵/3 ml in a medium of X-VIVO10 supplemented with 1% deionized bovine serum albumin, divalent iron-transferrin 500 µg/ml, 2% human AB type serum, SCF 100 ng/ml, IL-3 10 ng/ml and EPO 4 U/ml on a 6-well plate. Then liquid culture was performed for 14 days. On day 21, 3 ml of a medium with a composition the same as that described above was added. In the 3rd phase (days 28 to 31), the cells were suspended at a concentration of 1x10⁶/3 ml in a medium of X-VIVO10 supplemented with 1% deionized bovine serum albumin, divalent iron-transferrin 500 µg/ml, 2% human type AB serum and EPO 4 U/ml on a 6-well plate. The cells were cocultured with macrophages for 3 days. Macrophages used herein were derived from healthy human peripheral blood monocytes. Specifically, on day 21, peripheral blood was collected from a healthy individual, and monocytes were separated using a Rosette SepTM Antibody Cocktail (StemCell Technologies). The monocytes were suspended at a concentration of 3x10⁵/3 ml in IMDM supplemented with 2% human type AB serum and macrophage colony stimulating factor (M-CSF) 100 ng/ml, and then cultured for 7 days, so as to cause the monocytes to differentiate into macrophages. All of the cells were cultured under conditions of 37°C and 5% CO₂. The cells were collected at each phase, total cell number was counted, and then cell surface character was analyzed by flow cytometry. Further, cytospin samples were prepared, and May-Giemsa staining was performed, so that the cell morphology was observed.

### (2) Results:

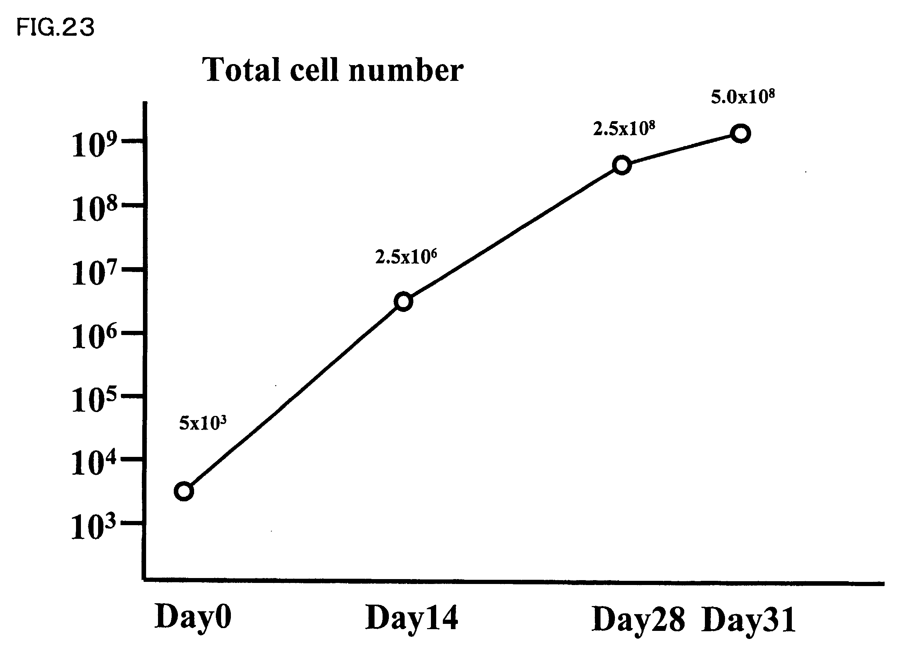

Figure 23 shows the total cell number obtained by culturing. By the coculture in the 1st phase, the total cell number was amplified 500-fold from 5x10³ to 2.5x10⁶. Further, by the liquid culture in the 2nd phase, the total cell number was amplified 50,000-fold to 2.5x10⁸, and in the 3rd phase, it was further amplified 100,000-fold to 5.0x10⁸.

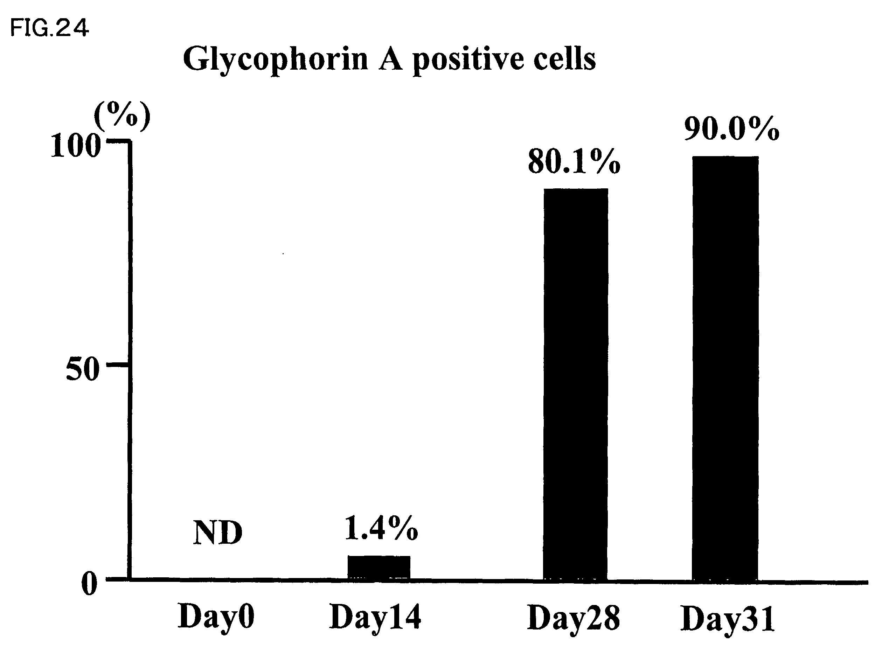

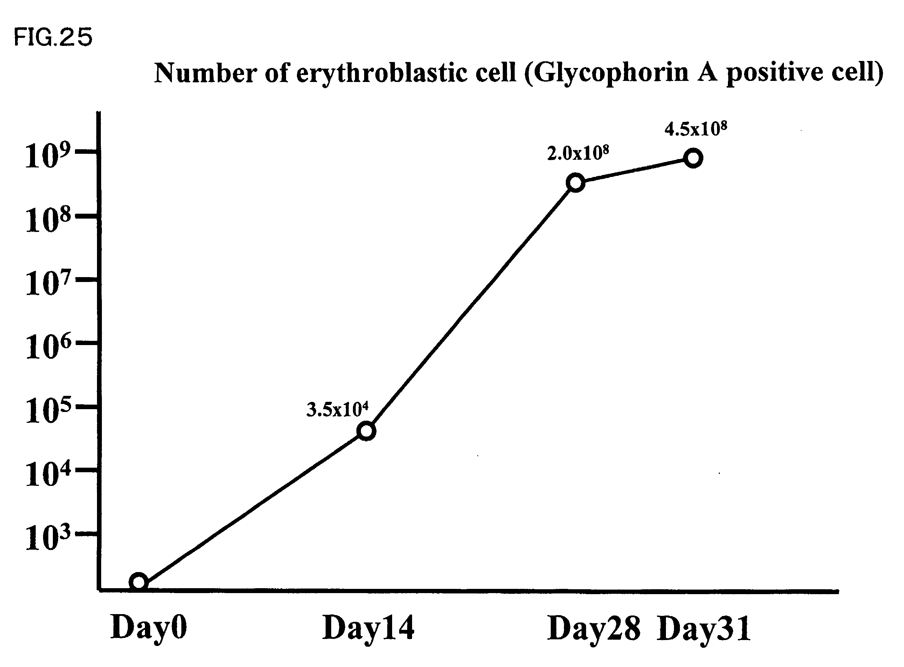

Figure 24 shows the results of analysis using flow cytometry. Although on day 14, Glycophorin positive cells merely accounted for 1.4%, the cells accounted for 80.1% on day 28 and 90% on day 31. That is, 5x10³ CD34 positive cells differentiated and expanded successfully to 2.0x10⁸ and 4.5x10⁸ erythroblastic cells on days 28 and 31, respectively (Fig. 25).

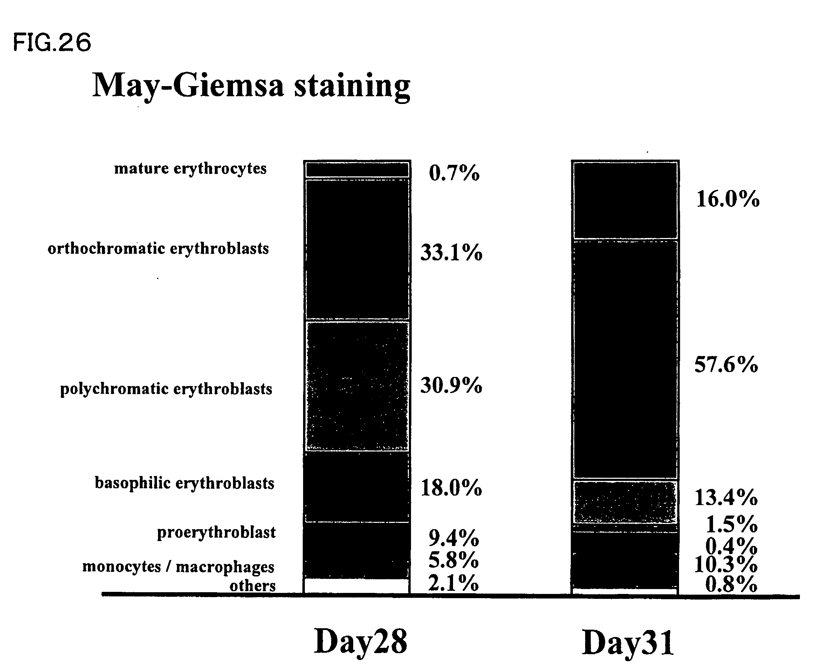

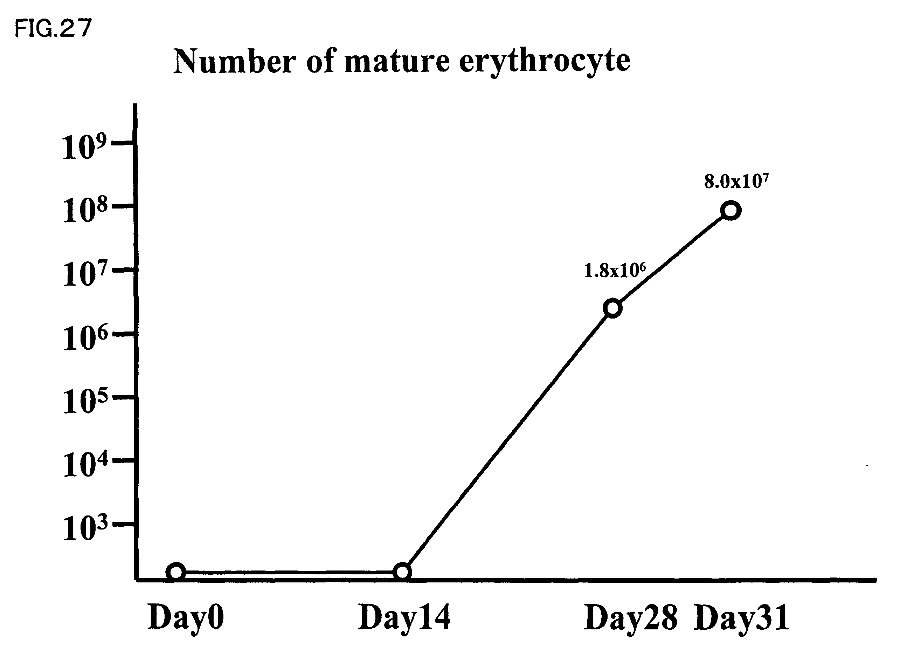

Figure 26 shows the results of May-Giemsa staining. On day 28, mature erythrocytes accounted for only 0.7%. However, erythroblasts were enucleated by 3 days of coculture with macrophages until day 31, and mature erythrocytes increased to 16%. Therefore, 5x10³ CD34 positive cells differentiated and expanded successfully to 1.8x10⁶ and 8.0x10⁷ mature erythrocytes on days 28 and 31, respectively (Fig. 27).

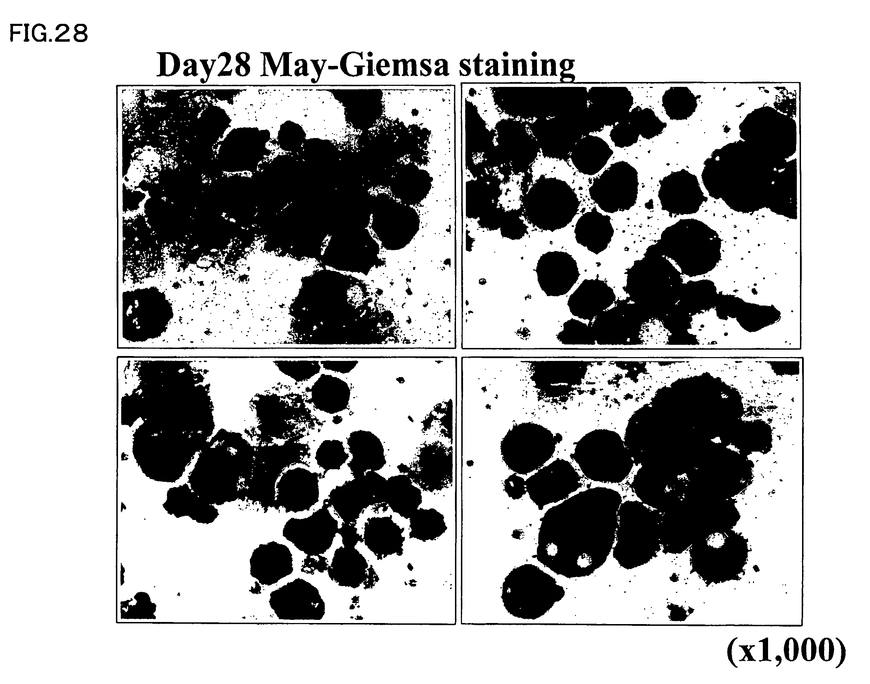

Figure 28 shows images of May-Giemsa staining on day 28. Most cells were erythroblastic cells, mainly comprising basophilic erythroblasts and polychromatic erythroblasts.

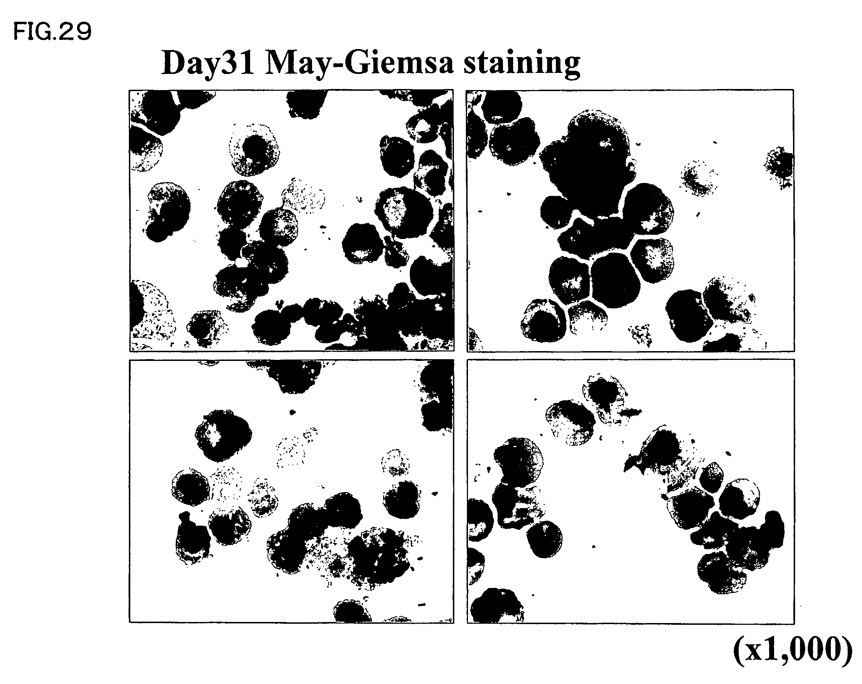

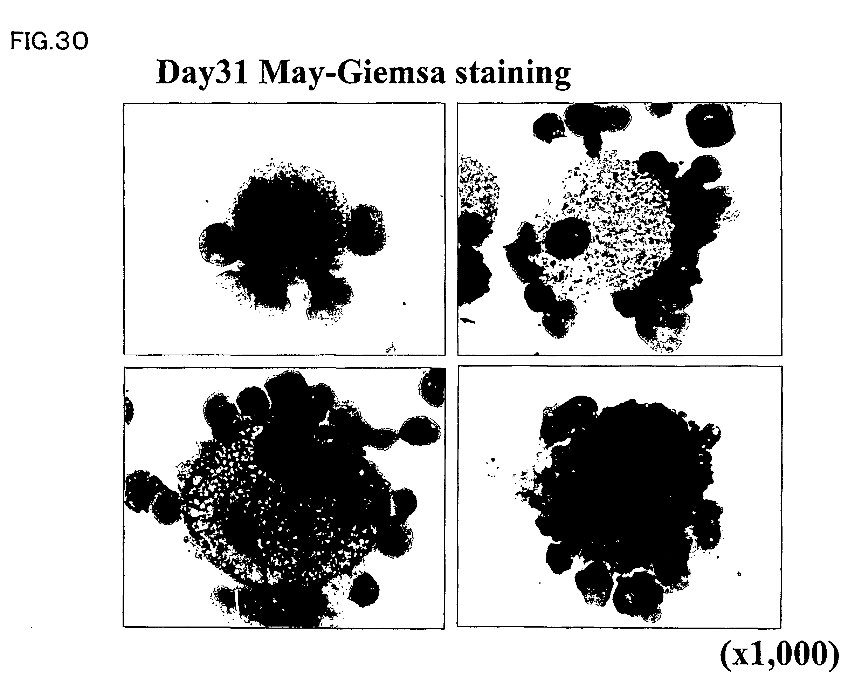

Figures 29 and 30 show images of May-Giemsa staining on day 31. Most cells were polychromatic erythroblasts, and many mature and enucleated erythrocytes were observed (Fig. 29). Erythroblastic cells were present surrounding a macrophage, forming the so-called Erythroblastic Island (Fig. 30).

It was confirmed by the above results that efficient differentiation and expansion of cord blood-derived CD34 positive cells into erythroblastic cells are possible by allowing the cells to expand by 14 days of coculture with immortalized stromal cells (hTERT stromal cell) followed by a further 14 days of liquid culturing in the presence of cytokines containing erythropoietin (EPO), and that efficient production of mature erythrocytes is possible by 3 days of coculture of the obtained erythroblastic cells with macrophages to enucleate the erythroblasts.

### [Example 9]

### (1) Transplantation into NOD/SCID (nonobese diabetic/severe combined immunodeficiency) mice

The mice used for transplantation were 6 to 10-week-old NOD/LtSz-scid (NOD/SCID) mice bred from breeding parents that had been obtained from LShultz (Jackson Laboratory, bay Harbor, ME, USA). All the mice were treated under sterilization, and kept in microisolators. In the presence of TPO, SCF and FL, a primary stromal cell layer or an hTERT-stromal cell layer was cocultured with CB CD34+ cells for 2 to 4 weeks. All the hematopoietic cells (HPCs) that had expanded above and beneath the stromal cell layer were collected. The contamination rate of stromal cells in hematopoietic cells was 0.01% or less under a microscope. Stromal cells can be easily distinguished from hematopoietic cells based on cell size and morphological features under a microscope. The obtained hematopoietic cells were injected via the lateral tail vein of mice irradiated with a dose of 400 cGy. Mononuclear cells were collected from the peripheral blood of a normal volunteer. 5x10⁶ mononuclear cells were then irradiated with a dose of 1500 cGy, and then cocultured as accessory cells with hematopoietic cells.

### (2) Study of engraftment of transplanted cells

The mice were sacrificed by cervical dislocation 6 weeks after transplantation, and then the bone marrow and peripheral blood mononuclear cells (as was reported previously) were collected. The presence of human hematopoietic cells was quantitatively determined by (i) detecting using flow cytometry cells that were stained by FITC-anti-human CD45 conjugates, and (ii) detecting a human genome ALU repetitive sequence gene DNA as described below.

### Detection of gene of human ALU repetitive sequence

Genomic DNA was isolated from the bone marrow and peripheral blood mononuclear cells of the transplanted NOD/SCID mice.

As primer sequences, 5'-CACCTGTAATCCCAGCAGTTT-3' and 5'-CGCGATCTCGGCTCACTGCA-3' were used. After being denatured at 94°C for 4 minutes, DNA samples were amplified by repeating 35 times a cycle consisting of 94°C for 1 minute (denaturation), 55°C for 45 seconds (annealing) and 72°C for 1 minute (extension).

The amplified products were visualized by ethidium bromide staining on 2.5% agarose gel electrophoresis as a 221 bp band.

### (3) Results

### Expansion of SRC (severe combined immunodeficiency (SCID) repopulating cells, SCID-repopulating cells)

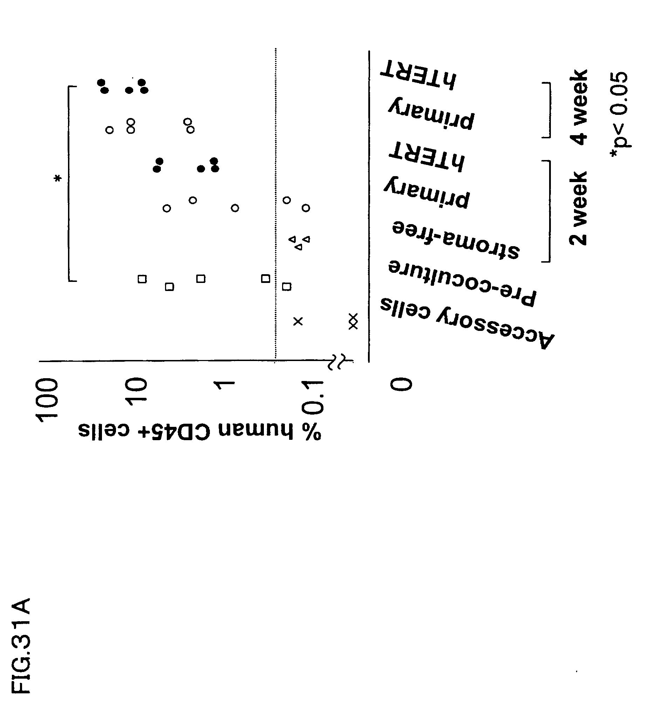

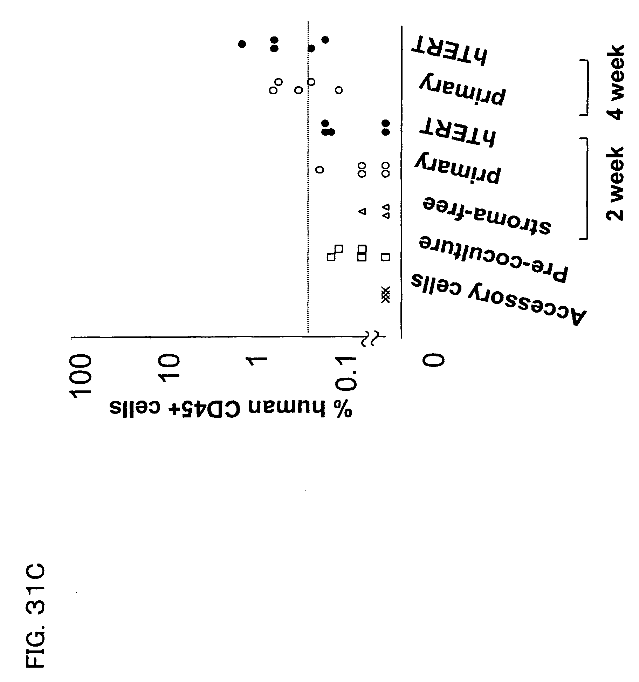

As a substitute for the in vivo human stem cell assay used to evaluate the expansion of HSCs (Hematopoietic stem cells, human hematopoietic stem cells), the engraftment of SRC was examined. The pre-cocultured cord blood CD34+ cells or the total expanded HPCs that had been generated from 2 or 4 weeks of coculture with each stromal cell line were transplanted into irradiated NOD/SCID mice. Simultaneously, irradiated, 5x10⁶ peripheral blood mononuclear cells were co-transplanted to roughly adjust the total number of the transplanted cells. Human cells in the bone marrow and peripheral blood of NOD/SCID mice were evaluated by flow cytometry and ALU PCR 6 weeks after transplantation (Fig. 31). It was found that when human cells (hCD45+) accounted for 0.13% or more, human ALU sequence could be detected by PCR amplification (Fig. 31 B, lanes 5 to 8), but the sequence could not be detected in the case of 0.07% hCD45+ (Fig. 31B, lane 4). Therefore, the detection limit for the human cells in the NOD/SCID mice was determined as 0.1 % hCD45+. hCD45+ cells were detected in the bone marrow of the mice, into which hematopoietic cells (previously subjected to 2 weeks of coculture with primary stromal cells or hTERT-stromal cells) had been transplanted (Fig. 31A). However, there was no significant difference in percentage % of hCD45+ cells between the mice transplanted with hematopoietic cells that had been pre-cocultured and cocultured with the primary stromal cells or hTERT-stromal cells. These results suggest that repopulating cells (SRCs) in NOD/SCID mice did not expand in 2 weeks, in spite of a significant increase in clonogenic cells (Table 2). This may be due to the quiescent nature of human hematopoietic stem cells. hCD45+ cells were not detected in the bone marrow of the mice, into which hematopoietic cells (previously cultured with cytokines for only 2 weeks) had been transplanted. These results suggest that stromal cells are required for maintaining SRA (SCID repopulating activity) of human hematopoietic cells, and SRA of the hematopoietic stem cells that were cocultured with hTERT-stromal cells may be maintained at the same level as that of the SRA of hematopoietic stem cells that were cocultured with primary stromal cells. hCD45+ cells were also detected in the bone marrow and peripheral blood of mice, into which hematopoietic cells (previously cocultured with stromal cells for 4 weeks) had been transplanted (Fig. 31A, 31C and 31D). The proportion of hCD45+ cells in the bone marrow of the mice, into which hematopoietic cells (previously cocultured with hTERT-stromal cells) had been transplanted, was significantly higher than that of hCD45+ cells in the bone marrow of mice, into which cells (pre-cocultured with CD34+ cells) had been transplanted. However, the proportion of hCD45+ cells in the bone marrow of the mice, into which hematopoietic cells (pre-cocultured with primary stromal cells) had been transplanted, was observed to tend to be higher than that of the mice, into which pre-cocultured CD34+ cells had been transplanted (p=0.07) (Table 3). These results suggest that amplification of SRC in cord blood CD34+ cells that had been cocultured with hTERT-stromal cells may be equivalent or superior to that of the case of coculture with primary stromal cells.

Next, surface markers of hematopoietic cells differentiating from SRC that had expanded on hTERT-stromal cells were tested (Fig. 32B). Most cells were CD19+ B-lymphocytic cells and CD11 myeloid lineage cells, and fewer cells were CD41+ and glycophorin A+ cells. The expression pattern of the surface antigens of the hematopoietic cells that had been cocultured with hTERT-stromal cells was the same as that in the case of hematopoietic cells cocultured with primary stromal cells (Fig. 32A) or the case of pre-cultured CD34+ cells (Fig. 32C). These results suggest that all of the hematopoietic precursor cells that have expanded on primary stromal cells or hTERT-stromal cells maintain equivalent pluripotency .

**Table 2**

| Ex vivo expansion of cord blood CD34+ cells (2 weeks) | | | |
|---|---|---|---|
| | Stromal cell-free | Primary stromal cells | hTERT-stromal cells |
| Total cell number | 64 ± 4 | 550 ± 25* | 550 ± 62* |
| CD34+ cell | 10 ± 2 | 117 ± 13* | 118 ± 8* |
| CFU-C | 6 ± 1 | 67 ± 5* | 71 ± 5* |
| CFU-Mix | 5 ± 0 | 52 ± 7* | 79 ± 36* |

| | | | |
|---|---|---|---|
| Figures are multiples of the initial number of cells. *P<0.05 vs. stroma free (n=4) (Student's *t* test). CFU-C indicates Colony-Forming Units in culture, and CFU-Mix indicates Colony-Forming Unit mixed cells. | | | |

The results are represented by mean value ± standard deviation (n=4).

**Table 3**

| Engraftment% of human CD45+ cells in bone marrow and peripheral blood of NOD/SCID mouse | | | |
|---|---|---|---|
| | Number of transplanted and re-constituted mice | CD45+ cell% in bone marrow of transplanted mouse | CD45+ cell% in peripheral blood of transplanted mouse |
| Accessory cells | 0/3 | n.d. | n.d. |
| Pre-coculture | 4/5 | 1.67 ± 2.06 | n.d. |
| Stromal cell-free (2 weeks) | 0/3 | n.d. | n.d. |
| Primary stromal cells (2 weeks) | 3/5 | 0.79 ± 1.00 | n.d. |
| hTERT (2 weeks) | 5/5 | 1.65 ± 1.33 | n.d. |
| Primary stromal cells (4 weeks) | 5/5 | 5.88 ± 4.86 | 0.15 ± 0.09 |
| hTERT (4 weeks) | 5/5 | 9.67 ± 5.57* | 0.27 ± 0.23 |

| | | | |
|---|---|---|---|
| *P<0.05, compared with a pre-cocultured group (Mann-Whitney U test). n.d. shows that hCD45 % was lower than the cut-off value (0.1 %). | | | |

### [Reference example]

### Differentiation from mesenchymal stem cells into hepatocytes

### (1) Method

Mesenchymal stem cells (MSC) were prepared by the method described in Example 5. The mesenchymal stem cells obtained from the primary culture were prepared and subcultured by the method described in Example 5, and then were used in the following experiment at PD4 (at the time period after 4 instances of population doubling) from the primary culture.

Spragne-Dawley (SD) rats (approximately 150 g, 5-week-old male) purchased from CHARLES RIVER JAPAN, INC., were used.

### Preparation of hepatopathy model and transplantation of MSC into liver

From the day before the hepatectomy (day -1), intraperitoneal administration of 10 mg/kg/day of cyclosporin A (CyA, Sandimmun: purchased from Novartis Pharm) to rats was begun. On day 0, partial (2/3) hepatectomy was performed according to a standard method (M. Brues et al J. Exp. Med. 65: 15, 1937), and then MSC (2x10⁶/300 µl) was locally injected using a 23G syringe to the remaining caudate lobe of the liver.

### (2) Study of differentiation into hepatocyte

On day 10 after hepatectomy, the rats were sacrificed. The liver was fixed by perfusion with 3% paraformaldehyde and embedded in an OTC compound to prepare frozen sections (6 µm). Then the sections were immuno-stained with an anti-human albumin antibody (Sigma, A6684), anti-human AFP antibody (Sigma, A8452), and anti-human CK19 antibody (Sigma, C6930).

### (3) Results

On day 10 after locally injecting MSC at PD4 into the remaining liver after the partial (2/3) hepatectomy, the rats were sacrificed. The liver locally injected with MSC cells was excised, fixed, and then stained using an anti-human albumin antibody (Sigma, A6684). The presence of cells showing the expression of human albumin was confirmed in the liver tissue of the normal rat (Fig. 34).

These examples demonstrated that under conditions and environment that are sufficiently appropriate for inducing differentiation into liver, such as within the liver tissue of an animal subjected to partial hepatectomy, human mesenchymal stem cells can be efficiently differentiated into mature hepatocytes.

### Industrial applicability

According to the present invention, a number of mesenchymal stem cells or mesenchymal cells that have conventionally been obtained in extremely a small number can be prepared, and mesenchymal cells can be differentiated into various cells. Thus, it becomes possible to implement various tests or therapies that have previously been unable to be performed, because of conventional inavailability of sufficient amount of cells.

For example, the above examples demonstrated that erythroblasts can be collected in large quantities by amplifying stem cells using TERT stromal cells, and then inducing the differentiation using SCF and EPO. Normally, the number of CD34 positive cells obtained from one umbilical cord is as few as approximately 1×10⁵. However, according to the present invention, 0.5 to 1×10⁶ erythroblasts can be produced from 5000 CD34 positive cells. Hence, from 1×10⁵ CD34 positive cells, a yield of around 1 to 2×10⁷ erythroblastic precursor cells can be expected every 2 weeks. This yield is far higher than that obtained from conventional methods. If a sufficient amount of cord blood can be supplied by the method of the present invention, the product obtained by this method has hidden potential to be a new source for blood transfusions. Therefore the present invention is very useful.

This description and the drawings include by reference the contents as disclosed in the description and the drawings of Japanese Patent Application No. 2001-335375 filed with the Japanese Patent Office on October 31, 2001, which is a priority document of the present application.

## Claims

1. An immortalized mesenchymal system-related cell, which is a mesenchymal system-related cell that is selected from mesenchymal stem cells, mesenchymal precursor cells, mesenchymal cells and cells derived from mesenchymal cells, and is immortalized by high expression or activation of an immortalizing gene.

2. The cell according to claim 1, wherein the immortalizing gene is any one of a telomerase gene, a gene derived from telomerase, and a gene regulating the expression or activity of telomerase.

3. The cell according to claim 1 or 2, wherein the immortalizing gene can be specifically deleted or is already deleted by gene deletion.

4. The cell according to claim 3, wherein the deletion of the immortalizing gene is performed by placing the immortalizing gene between loxP sequences or loxP-like sequences, and then treating the gene with a recombinase such as Cre recombinase.

5. The cell according to claim 1, wherein the immortalizing gene is highly expressed by introducing the immortalizing gene into a cell by gene introduction.

6. The cell according to any one of claims 1 to 5, wherein the mesenchymal system-related cell that is selected from mesenchymal stem cells, mesenchymal precursor cells, mesenchymal cells and cells derived from mesenchymal cells is derived from any one of bone marrow, peripheral blood, cord blood, skin, hair root and muscle tissue.

7. The cell according to any one of claims 1 to 6, wherein the mesenchymal system-related cell that is selected from mesenchymal stem cells, mesenchymal precursor cells, mesenchymal cells and cells derived from mesenchymal cells is derived from either human ES cells (fetal stem cells) or cells derived from ES cells (fetal stem cells).

8. The cell according to any one of claims 1 to 7, wherein the mesenchymal system-related cell is a mesenchymal cell.

9. The cell according to claim 8, wherein the mesenchymal cell is a bone marrow stromal cell that can support the growth of hematopoietic system cells.

10. The cell according to any one of claims 1 to 7, wherein the mesenchymal system-related cell is a cell derived from mesenchymal cells.

11. The cell according to claim 10, wherein the cell derived from mesenchymal cells is a cell of the cardiovascular system such as an endothelial cell or cardiac muscle cell, or a precursor cell of the cells of the cardiovascular system.

12. The cell according to claim 10, wherein the cell derived from mesenchymal cells is a cell or a precursor cell of any one of bone, cartilage, tendon, skeletal muscle and adipose tissue.

13. The cell according to claim 10, wherein the cell derived from mesenchymal cells is a. nervous system cell or a precursor cell of a nervous system cell.

14. The cell according to claim 10, wherein the cell derived from mesenchymal cells is an endocrine cell or a precursor cell of an endocrine cell.

15. The cell according to claim 10, wherein the cell derived from mesenchymal cells is a hematopoietic cell or a precursor cell of a hematopoietic cell.

16. The cell according to claim 10, wherein the cell derived from mesenchymal cells is a hepatocyte or a precursor cell of a hepatocyte.

17. Artificial bone marrow having differentiation potency and expansion potency, which is obtained by culturing the cells of claim 9 with hematopoietic system stem cells.

18. The artificial bone marrow according to claim 17, which is obtained by further allowing the presence of immortalized stromal cells or a substance derived from the immortalized stromal cells.

19. The artificial bone marrow according to claim 18, wherein the substance derived from the immortalized stromal cells is a substance that is selected from soluble cytokines contained in a conditioned medium, adhesive molecules supplied by the contact with cells, and insoluble cytokine ligands.

20. The artificial bone marrow according to claims 17 to 19, which contains erythrocytes or precursor cells of erythrocytes.

21. The artificial bone marrow according to claims 17 to 19, which contains blood platelets or precursor cells of blood platelets such as megakaryocytes.

22. An artificial cell construct of the cardiovascular system, which can be obtained by culturing the precursor cells of cardiovascular cells together with the cell of any one of claims 1 to 7, and 11, has expansion potency, and whose differentiation can be regulated.

23. Artificial bone, artificial cartilage, artificial tendon, artificial skeletal muscle or artificial adipose tissue, which can be obtained by culturing cells having properties of the precursor cells of the cells of any one of bone, cartilage, tendon, skeletal muscle and adipose tissue together with the cell of any one of claims 1 to 7, and 12, has expansion potency, and whose differentiation can be regulated.

24. A cell group that is part of the nervous system or a cell group co-existing in the nervous system, which can be obtained by culturing the precursor cells of the nervous system together with the cell of any one of claims 1 to 7, and 13, or substances derived from these cells, and whose expansion or differentiation can be regulated.

25. A cell group that is part of the endocrine tissue or a cell group coexisting in the endocrine tissue, which can be obtained by culturing the precursor cells of the endocrine system together with the cell of any one of claims 1 to 7, and 14, or with substances derived from these cells, and whose expansion or differentiation can be regulated.

26. A nerve stem cell, which is induced from the cell of claim 8 by differentiation-inducing treatment.

27. An examination method, which is for evaluating the drug efficacy of a neuroactive . agent using the cell of claim 8.

28. A bone marrow stromal cell, which is made to differentiate from the cell of claim 8 by differentiation-inducing treatment.

29. An examination method for evaluating the drug efficacy of an agent, which uses the cell of claim 28.

30. An erythrocyte, which is induced by differentiating the artificial bone marrow of claim 17.

31. A method for immortalizing and proliferating mesenchymal system-related cells, which comprises causing high expression of or activating an immortalizing gene in a mesenchymal system-related cell that is selected from mesenchymal precursor cells, mesenchymal stem cells, mesenchymal cells and cells derived from mesenchymal cells.

32. The method according to claim 31, wherein the immortalizing gene is a telomerase.

33. The method according to claim 31, wherein high expression is caused by introducing telomerase using a retrovirus vector into a cell derived from mesenchymal stem cells.

34. The method according to claim 33, wherein the retrovirus vector is pBabe.

35. The method according to claim 31, wherein an oncogene is not introduced into a cell derived from mesenchymal stem cells.

36. The method according to any one of claims 31 to 35, wherein the mesenchymal system-related cell is a bone marrow stromal cell.

37. A method for producing artificial bone marrow, which comprises culturing immortalized and expanded bone marrow stromal cells together with hematopoietic stem cells.
